# EUROPEAN PATENT SPECIFICATION

(11) **EP 2 198 057 B1**
(45) Date of publication and mention of the grant of the patent: **18.05.2016**
(21) Application number: 08837537.3
(22) Date of filing: 07.10.2008
(51) Int. Cl.: C12Q 1/68, C07H 21/00

(54) **DETECTION OF ATYPICAL PNEUMONIA**
NACHWEIS VON ATYPISCHER PNEUMONIE
DÉTECTION D'UNE PNEUMONIE ATYPIQUE

(30) Priority: 09.10.2007 US 869650
(43) Date of publication of application: 23.06.2010
(73) Proprietor: Quest Diagnostics Investments Incorporated, Wilmington, DE 19899 (US)
(72) Inventor: AYE, Michael, Fountain Valley California 92708 (US); LEE, Ming-Chou, Mission Viejo California 92692 (US); KONG, Lilly I., Covina California 91724 (US); TABB, Michelle M., Santa Ana California 92705 (US); CHEN, Fan, Fullerton California 92831 (US); CHEN, Jules, Walnut California 91789 (US)
(74) Representative: Schiweck, Weinzierl & Koch
(86) International application number: PCT/US2008/079080
(87) International publication number: WO 2009/048873

(56) References cited:
- EP-A1- 0 943 691
- DE-A1- 10 115 748
- US-A1- 2006 210 967
- US-B1- 6 277 582
- US-B1- 6 822 071
- PITCHER DAVID ET AL: "Real-time detection of Mycoplasma pneumoniae in respiratory samples with an internal processing control", JOURNAL OF MEDICAL MICROBIOLOGY, vol. 55, no. 2, February 2006 (2006-02), pages 149-155, XP002628169, ISSN: 0022-2615
- STRALIN KRISTOFFER ET AL: "Design of a multiplex PCR for Streptococcus pneumoniae, Haemophilus influenzae, Mycoplasma pneumoniae and Chlamydophila pneumoniae to be used on sputum samples", APMIS, vol. 113, no. 2, February 2005 (2005-02), pages 99-111, XP002628170, ISSN: 0903-4641
- CAMPBELL ET AL.: 'Evaluation of Chlamydia pneumoniae 43- and 53-Kilodalton Recombinant Proteins for Serodiagnosis by Western Blot. Clinial and Diagnostic Laboratory Immunology' vol. 8, no. 6, November 2001, pages 1231 - 1233, XP002516777
- MIYAKE ET AL.: 'Characterization of Legionella pneumophila pmiA, a Gene Essential for Infectivity of Protozoa and Macrophages.' INFECTION AND IMMUNITY vol. 73, no. 10, October 2005, pages 6272 - 6282, XP008132390
- DATABASE EMBL 21 October 2004 MIYAKE ET AL., XP008138589 Database accession no. AB193439
- DALLO S F ET AL: "DNA AND PROTEIN SEQUENCE HOMOLOGIES BETWEEN THE ADHESINS OF MYCOPLASMA GENITALIUM AND MYCOPLASMA PNEUMONIAE", INFECTION AND IMMUNITY, AMERICAN SOCIETY FOR MICROBIOLOGY, US, vol. 57, no. 4, 1 April 1989 (1989-04-01), pages 1059-1065, XP001013310, ISSN: 0019-9567
- PITCHER D G ET AL: 'Mycoplasma amphoriforme sp. nov., isolated from a patient with chronic bronchopneumonia.' INTERNATIONAL JOURNAL OF SYSTEMATIC AND EVOLUTIONARY MICROBIOLOGY NOV 2005 vol. 55, no. PT 6, November 2005, pages 2589 - 2594 ISSN: 1466-5026
- CIMOLAI N ET AL: 'Immunological cross-reactivity of a Mycoplasma pneumoniae membrane-associated protein antigen with Mycoplasma genitalium and Acholeplasma laidlawii.' JOURNAL OF CLINICAL MICROBIOLOGY vol. 25, no. 11, November 1987, pages 2136 - 2139 ISSN: 0095-1137
- CHERNESKY MAX A: 'The laboratory diagnosis of Chlamydia trachomatis infections.' THE CANADIAN JOURNAL OF INFECTIOUS DISEASES & MEDICAL MICROBIOLOGY = JOURNAL CANADIEN DES MALADIES INFECTIEUSES ET DE LA MICROBIOLOGIE MÉDICALE / AMMI CANADA JAN 2005 vol. 16, no. 1, January 2005, pages 39 - 44 ISSN: 1712-9532
- BASEMAN J B ET AL: "ISOLATION AND CHARACTERIZATION OF MYCOPLASMA-GENITALIUM STRAINS FROM THE HUMAN RESPIRATORY TRACT", JOURNAL OF CLINICAL MICROBIOLOGY, vol. 26, no. 11, 1988, pages 2266-2269, ISSN: 0095-1137

## Description

### FIELD OF INVENTION

This invention relates to the field of pathogen detection.

### BACKGROUND OF INVENTION

The following discussion of the background of the invention is merely provided to aid the reader in understanding the invention and is not admitted to describe or constitute prior art to the present invention.

Pneumonia is a lower respiratory tract infection characterized by inflammation of lung tissue. "Typical" pneumonia is usually diagnosed from a chest X-ray in which the lobar lung area is radio-opaque and is caused by *Streptococcus pneumoniae.* Less frequently, patients develop "atypical" pneumonia. This form of the disease is characterized by an abnormal or diffuse chest X-ray and is more difficult for the physician to diagnose. Atypical pneumonia is caused by less common bacterial pathogens including, for example, *Mycoplasma pneumoniae, Chlamydophila pneumoniae, Legionella pneumophila,* and certain viruses including the SARS corona virus.

The different types of atypical pneumonia are virtually impossible to distinguish based on clinical symptoms alone and are frequently confused with less serious medical conditions. It is therefore important for the clinician to have access to rapid and accurate diagnostic tests capable of detecting and distinguishing among the various pneumonia-causing organisms.

Bacterial culturing is one method for detecting pathogens. However, atypical bacteria are difficult to culture and the time required to obtain results is long. *M. pneumoniae* cultures from clinical samples may take 2-3 weeks, and the success rate depends on proper sample collection, prompt sample processing, and the expertise of the microbiology laboratory personnel. For *C. pneunioniae,* the cell culture success rate is low even for experience clinical microbiologists. *L. pneumophila* is the easiest of the three to culture, but it still takes 3-7 days for identification, and the sensitivity is poor. Campbell et al., Clinical and Diagnostic Laboratory Immunology (2001) 8, 6, 1231-1233 relates to the use of recombinant antigens in the diagnosis of *C*. *pneumoniae* infection. US 2006/210967 A1 relates to the detection and identification of a number of pathogens, including *M. pneumoniae, C. pneumoniae* and *Legionella.* EP 0 943 691 A1 discloses a multiplex assay for the simultaneous detection of at least two of the *L. pneurnophila* mip gene, the *M. pneumoniae* P1 gene and the *C*. *pneumoniae* OmpA gene in a clinical sample. US 6 277 582 B1 relates to a detection assay for detecting *M. pneumoniae* by detecting the P1 gene using various primers and probes targeted to the *M. pneumoniae* P1 gene. Pitcher et al., Journal of Medical Microbiology (2006) 55, 2, 149-155 relates to real time detection of *M. pneumoniae* based on real time PCR. Kristoffer et al., APMIS (2005) 113, 2, 99-111 discloses a multiplex assay for detecting various pathogens including inter alia the *M. pneumoniae* P1 gene and the *C*. *pneumoniae* OmpA gene.

Serology encompasses the conventional methods of diagnosing atypical pneumonia, including ELISA-based assays, complement fixation, microparticle agglutination, and western blotting. However, these methods often require obtaining multiple biological samples from the patient, preferably at least two samples, one during the infectious state and one during the convalescent state. Accordingly, these assays have a high rate of false negative readings.

### SUMMARY OF THE INVENTION

The present invention provides compositions and methods for identifying pathogen in a biological sample and/or diagnosing an individual as having atypical pneumonia. Specifically, the present invention enables the detection of pathogenic bacteria known to cause atypical pneumonia by detecting the presence of bacterial nucleic acids in the sample. The detection methodology is based on the discovery of particular target sequences within the bacterial genomes which are useful indicators of bacterial infection.

The invention relates to a method for identifying the presence or absence of a pathogen in a biological sample, comprising detecting the presence or absence of a *Mycoplasma pneumoniae* P1 gene fragment comprising at least 15 nucleotides from the sequence of SEQ ID NO: 2, or a complement thereof.

The invention also relates to an *in nitro* method of diagnosing an individual for infection with an atypical pneumoniae organism, comprising evaluating a biological sample obtained from the individual for the presence or absence of a *Mycoplasma pneumoniae* P1 gene fragment comprising at least 15 nucleotides from the sequence of SEQ ID NO: 2, or a complement thereof, wherein the presence of said gene or fragment indicates that the individual is infected with the associated organism.

The invention also relates to a kit comprising: a pair of P1 primers that specifically hybridize to the P1 gene of *M. pneumoniae* and are capable of amplifying a P1 gene fragment, wherein the P1 primers specifically hybridize to a nucleic acid having the sequence of SEQ ID NO: 2; and a P1 probe capable of specifically hybridizing to the P1 fragment amplified by the P1 primers, wherein optionally the P1 probe specifically hybridizes to a nucleic acid having the sequence of SEQ ID NO: 2.

Disclosed in the context of the invention is a method for identifying a pathogen in a biological sample by detecting any two (or all three) of:
(a) the *Mycoplasma pneumoniae* P1 gene or fragment thereof,
(b) the *Chlamydophila pneumoniae* Cpn0980 gene or fragment thereof, and
(c) the *Legionella pneumophila* pmiA gene or fragment thereof,

In another aspect, the disclosure provides a method for diagnosing an individual for infection with an atypical pneumoniae organism, comprising evaluating a biological sample from the individual for the presence or absence of any two or more of:
(a) the *Mycoplasma pneumoniae* P1 gene or fragment thereof,
(b) the *Chlamydophila pneumoniae* Cpn0980 gene or fragment thereof, and
(c) the *Legionella pneumophila* pmiA gene or fragment thereof,
wherein the presence of said gene or fragment indicates that the individual is infected with the associated organism.

According to the disclosure, the method further comprises amplifying (e.g., by PCR) one, two, or all three of the genes and/or gene fragments. Preferably, amplification is performed in a single reaction (e.g., a multiplex reaction). More preferably, amplification and detection is performed in a single reaction.

The invention also provides an *in nitro* method for identifying a pathogen in a biological sample by:
(a) providing oligonucleotide primer pairs suitable for amplifying, in a single reaction:
   (i) *the Mycoplasma pneumoniae* P1 gene or a fragment thereof, and preferably at least one of: (ii) the *Chlamydophila pneumoniae* Cpn0980 gene or a fragment thereof, and (iii) the *Legionella pneumophila* pmiA gene or a fragment thereof, wherein each of the amplified genes and/or fragments is at least 15 nucleotides in length;
(b) contacting the biological sample with the primer pairs of step (a) under conditions wherein amplification products are produced, and
(c) identifying a pathogen by detecting the amplification products produced in step (b).

In preferred embodiments, the P1 gene fragment is a nucleic acid having at least 15 nucleotides that are substantially identical to the sequence of SEQ ID NO: 2, or a complement thereof. Preferably, the P1 gene fragment contains the sequence of SEQ ID NO: 3, or a complement thereof and/or is amplified using one or more primers containing the sequence of SEQ ID NOs: 4-5, or complements thereof. In other preferred embodiments, the P1 gene or gene fragment is detected using an oligonucleotide probe containing the sequence of SEQ ID NO: 3, or a complement thereof.

In other preferred embodiments, the Cpn0980 gene fragment is a nucleic acid having at least 15 nucleotides that are substantially identical to the sequence of SEQ ID NO: 7, or a complement thereof. Preferably, the Cpn0980 gene fragment contains the sequence of SEQ ID NO: 8, or a complement thereof and/or is amplified using one or more primers containing the sequence of SEQ ID NOs: 9-10, or complements thereof. In other preferred embodiments, the Cpn0980 gene or gene fragment is detected using an oligonucleotide probe containing the sequence of SEQ ID NO: 8, or a complement thereof.

In other preferred embodiments, the pmiA gene fragment is a nucleic acid having at least 15 nucleotides that are substantially identical to the sequence of SEQ ID NO: 12, or a complement thereof. Preferably, the pmiA gene fragment contains the sequence of SEQ ID NO: 13, or a complement thereof and/or is amplified using one or more primers containing the sequence of SEQ ID NOs: 14-17, or complements thereof. In other preferred embodiments, the pmiA gene or gene fragment is detected using an oligonucleotide probe containing the sequence of SEQ ID NO: 13, or a complement thereof.

In other preferred embodiments, each of the P1 gene or gene fragment, Cpn0980 gene or gene fragment, and pmiA gene or gene fragment is detected. Preferably, the genes and/or fragments are detected simultaneously, and more preferably, in a multiplex real-time PCR assay. In other useful embodiments, the one or more of the amplification primers are Scorpion primers including, for example, primers having the sequence of SEQ ID NOs: 18-25 and 45-48, or complements thereof.

In other preferred embodiments, the gene fragments consist of at least 20 nucleotides (e.g., 25, 30, 35, 40, 50, 75, 100, 150, 200, 250, 500 nucleotides, or more) that have a nucleotide sequence that is substantially identical (or identical) to the nucleotide sequence of the reference gene.

It is recognized that any of the foregoing genes or gene fragments may be assayed individually to identify the individual pathogens, or may be assayed in combination with each other (e.g., any two or all three genes) and/or with other biological indicators (e.g., proteins, nucleic acids, antigens, etc.) for the same or different organisms. Furthermore, any of the foregoing methods, alone or in combination with clinical evaluation or other diagnostic methods (e.g, lung X-ray), may be used to diagnose an individual as having atypical pneumonia.

In another aspect, the disclosure provides target nucleic acids for pathogens capable of causing atypical pneumonia. Specifically, the disclosure provides isolated nucleic acids that are at least about 90% identical (e.g., about 95% identical, about 99% identical, or 100% identical) to at least 20 contiguous nucleotides (e.g., 25, 30, 35, 40, 50, 75, 100, 150, 200, 250, 500 nucleotides, or more) of SEQ ID NOs: 2, 7, or 12, or complements thereof.

In another aspect, the invention provides a kit containing:
(a) a pair of P1 primers that specifically hybridize to the P1 gene of *M. pneumoniae* and are capable of amplifying a P1 gene fragment, and a P1 probe capable of specifically hybridizing to the P1 fragment amplified by the P1 primers; and preferably at least one of:
   (b) a pair of Cpn0980 primers that specifically hybridize to the Cpn0980 gene of *C*. *pneumoniae* and are capable of amplifying a Cpn0980 gene fragment, and a Cpn0980 probe capable of specifically hybridizing to the Cpn0980 fragment amplified by the Cpn0980 primers; and
   (c) a pair of pmiA primers that specifically hybridize to the pmiA gene *of L. pneumophila* and are capable of amplifying a pmiA gene fragment, and a pmiA probe that specifically hybridizes to the pmiA gene fragment amplified by the pmiA primers.

In preferred embodiments, the P1 primers and/or probe specifically hybridize to a nucleic acid having the sequence of SEQ ID NO: 2. In other preferred embodiments, the Cpn0980 primers and/or probe specifically hybridize to a nucleic acid having the sequence of SEQ ID NO: 6. In other preferred embodiments, the pmiA primers and/or probe specifically hybridize to a nucleic acid having the sequence of SEQ ID NO: 11.

### BRIEF DESCRIPTION OF THE FIGURES

FIGURE 1 is the nucleotide sequence of the *M. pneumoniae* isolate Mp1842 cytadhesin P1 gene provided in GenBank Accession No. AF290002 (SEQ ID NO: 1).
FIGURE 2 is the nucleotide sequence of the *C*. *pneumoniae* Cpn0980 gene (SEQ ID NO: 6).
FIGURE 3 is the nucleotide sequence of the *L. pneumophila* pmiA gene provided in GenBank Accession No. AB193439 (SEQ ID NO: 11).

### DETAILED DESCRIPTION OF INVENTION

The present invention provides methods, compositions, and kits suitable for identifying pathogens capable of causing atypical pneumonia in a biological sample that may contain nucleic acids from those organisms. In particular, the invention is useful for detecting *Mycoplasma pneumoniae, Chlamydophila pneumoniae,* and/or *Legionella pneumophila.*

*M. pneumoniae* may be identified by detecting the P1 gene or a gene fragment thereof. *C*. *pneumoniae* may be identified by detecting the Cpn0980 gene or a gene fragment thereof. And, *L. pneumophila* may be identified by detecting the pmiA gene or a fragment thereof. In one aspect, the invention provides methods for identifying two or more pathogens simultaneously.

As used herein, unless otherwise stated, the singular forms "a," "an," and "the" include plural reference. Thus, for example, a reference to "an oligonucleotide" includes a plurality of oligonucleotide molecules, and a reference to "a nucleic acid" is a reference to one or more nucleic acids.

As used herein, "about" means plus or minus 10%.

The terms "amplification" or "amplify" as used herein includes methods for copying a target nucleic acid, thereby increasing the number of copies of a selected nucleic acid sequence. Amplification may be exponential or linear. A target nucleic acid may be either DNA or RNA. The sequences amplified in this manner form an "amplicon." While the exemplary methods described hereinafter relate to amplification using the polymerase chain reaction (PCR), numerous other methods are known in the art for amplification of nucleic acids (e.g., isothermal methods, rolling circle methods, etc.). The skilled artisan will understand that these other methods may be used either in place of, or together with, PCR methods. See, e.g., Saiki, "Amplification of Genomic DNA" in PCR Protocols, Innis et al., Eds., Academic Press, San Diego, CA 1990, pp 13-20; Wharam, et al., Nucleic Acids Res. 2001 Jun 1;29(11):E54-E54; Hafner, et al., Biotechniques 2001 Apr;30(4):852-6, 858, 860; Zhong, et al., Biotechniques 2001 Apr;30(4):852-6, 858, 860.

The term "complement" "complementary" or "complementarity" as used herein with reference to polynucleotides (i.e., a sequence of nucleotides such as an oligonucleotide or a target nucleic acid) refers to standard Watson/Crick pairing rules. The complement of a nucleic acid sequence such that the 5' end of one sequence is paired with the 3' end of the other, is in "antiparallel association." For example, the sequence "5'-A-G-T-3"' is complementary to the sequence "3'-T-C-A-5'." Certain bases not commonly found in natural nucleic acids may be included in the nucleic acids described herein; these include, for example, inosine, 7-deazaguanine, Locked Nucleic Acids (LNA), and Peptide Nucleic Acids (PNA). Complementarity need not be perfect; stable duplexes may contain mismatched base pairs, degenerative, or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. A complement sequence can also be a sequence of RNA complementary to the DNA sequence or its complement sequence, and can also be a cDNA. The term "substantially complementary" as used herein means that two sequences specifically hybridize (defined below). The skilled artisan will understand that substantially complementary sequences need not hybridize along their entire length.

As used herein, the term "substantially identical", when referring to a nucleic acid, is one that has at least 80%, 85%, 90%, 95%, or 99% sequence identify to a reference nucleic acid sequence. The length of comparison is preferably the full length of the nucleic acid, but is generally at least 20 nucleotides, 30 nucleotides, 40 nucleotides, 50 nucleotides, 75 nucleotides, 100 nucleotides, 125 nucleotides, or more.

As used herein, the term "detecting" used in context of detecting a signal from a detectable label to indicate the presence of a target nucleic acid in the sample does not require the method to provide 100% sensitivity and/or 100% specificity. As is well known, "sensitivity" is the probability that a test is positive, given that the person has a target nucleic acid sequence, while "specificity" is the probability that a test is negative, given that the person does not have the target nucleic acid sequence. A sensitivity of at least 50% is preferred, although sensitivities of at least 60%, at least 70%, at least 80%, at least 90% and at least 99% are clearly more preferred. A specificity of at least 50% is preferred, although sensitivities of at least 60%, at least 70%, at least 80%, at least 90% and at least 99% are clearly more preferred. Detecting also encompasses assays with false positives and false negatives. False negative rates may be 1%, 5%, 10%, 15%, 20% or even higher. False positive rates may be 1%, 5%, 10%, 15%, 20% or even higher.

A "fragment" in the context of a gene fragment refers to a sequence of nucleotide residues which are at least about 20 nucleotides, at least about 25 nucleotides, at least about 30 nucleotides, at least about 40 nucleotides, at least about 50 nucleotides, or at least about 100 nucleotides. The fragment is typically less than about 400 nucleotides, less than about 300 nucleotides, less than about 250 nucleotides, less than about 200 nucleotides, or less than 150 nucleotides. In certain embodiments, the fragments can be used in various hybridization procedures or microarray procedures to identify specific pathogens.

By "isolated", when referring to an oligonucleotide, protein, polypeptide and the like, is meant one that has been separated from the components that naturally accompany it. For example, an oligonucleotide is substantially pure when it is removed from the genome of the organism from which it is derived. Typically, a substance is isolated when it is at least 50%, 75%, 85%, 90%, 95%, or even 99%, by weight, free from the biological molecules with which it is naturally present.

Oligonucleotides used as primers or probes for specifically amplifying (i.e., amplifying a particular target nucleic acid sequence) or specifically detecting (i.e., detecting a particular target nucleic acid sequence) a target nucleic acid generally are capable of specifically hybridizing to the target nucleic acid.

The term "multiplex PCR" as used herein refers to simultaneous amplification of two or more products within the same reaction vessel. Each product is primed using a distinct primer pair. A multiplex reaction may further include specific probes for each product, that are detectably labeled with different detectable moieties.

As used herein, the term "oligonucleotide" refers to a short polymer composed of deoxyribonucleotides, ribonucleotides or any combination thereof. Oligonucleotides are generally between about 10, 11, 12, 13, 14 or 15 to about 150 nucleotides (nt) in length, more preferably about 10, 11, 12, 13, 14, or 15 to about 70 nt, and most preferably between about 18 to about 26 nt in length. The single letter code for nucleotides is as described in the U.S. Patent Office Manual of Patent Examining Procedure, section 2422, table 1. In this regard, the nucleotide designation "R" means purine such as guanine or adenine, "Y" means pyrimidine such as cytosine or thymidine (uracil if RNA); "M" means adenine or cytosine, and "S" means guanine or cytosine. An oligonucleotide may be used as a primer or as a probe.

By "pathogen" is meant any microbial organism capable of causing atypical pneumonia in a mammal (e.g., a human). Specific pathogens include, for example, *Mycoplasma pneumoniae, Chlamydophila pneumoniae,* and *Legionella pneumophila.*

As used herein, a "primer" for amplification is an oligonucleotide that is complementary to a target nucleotide sequence and leads to addition of nucleotides to the 3' end of the primer in the presence of a DNA or RNA polymerase. The 3' nucleotide of the primer should generally be identical to the target sequence at a corresponding nucleotide position for optimal expression and amplification. The term "primer" as used herein includes all forms of primers that may be synthesized including peptide nucleic acid primers, locked nucleic acid primers, phosphorothioate modified primers, labeled primers, and the like. As used herein, a "forward primer" is a primer that is complementary to the anti-sense strand of dsDNA. A "reverse primer" is complementary to the sense-strand of dsDNA.

Primers are typically between about 10 and about 100 nucleotides in length, preferably between about 15 and about 60 nucleotides in length, and most preferably between about 25 and about 40 nucleotides in length. There is no standard length for optimal hybridization or polymerase chain reaction amplification. An optimal length for a particular primer application may be readily determined in the manner described in H. Erlich, PCR Technology, Principles and Application for DNA Amplification, (1989).

An oligonucleotide (e.g., a probe or a primer) that is specific for a target nucleic acid will "hybridize" to the target nucleic acid under suitable conditions. As used herein, "hybridization" or "hybridizing" refers to the process by which an oligonucleotide single strand anneals with a complementary strand through base pairing under defined hybridization conditions.

"Specific hybridization" is an indication that two nucleic acid sequences share a high degree of complementarity. Specific hybridization complexes form under permissive annealing conditions and remain hybridized after any subsequent washing steps. Permissive conditions for annealing of nucleic acid sequences are routinely determinable by one of ordinary skill in the art and may occur, for example, at 65°C in the presence of about 6xSSC. Stringency of hybridization may be expressed, in part, with reference to the temperature under which the wash steps are carried out. Such temperatures are typically selected to be about 5°C to 20°C lower than the thermal melting point (Tm) for the specific sequence at a defined ionic strength and pH. The Tm is the temperature (under defined ionic strength and pH) at which 50% of the target sequence hybridizes to a perfectly matched probe. Equations for calculating Tm and conditions for nucleic acid hybridization are known in the art.

As used herein, an oligonucleotide is "specific" for a nucleic acid if the oligonucleotide has at least 50% sequence identity with a portion of the nucleic acid when the oligonucleotide and the nucleic acid are aligned. An oligonucleotide that is specific for a nucleic acid is one that, under the appropriate hybridization or washing conditions, is capable of hybridizing to the target of interest and not substantially hybridizing to nucleic acids which are not of interest. Higher levels of sequence identity are preferred and include at least 75%, at least 80%, at least 85%, at least 90%, at least 95% and more preferably at least 98% sequence identity. Sequence identity can be determined using a commercially available computer program with a default setting that employs algorithms well known in the art. As used herein, sequences that have "high sequence identity" have identical nucleotides at least at about 50% of aligned nucleotide positions, preferably at least at about 60% of aligned nucleotide positions, and more preferably at least at about 75% of aligned nucleotide positions.

As used herein, the term "sample" or "biological sample" refers to clinical samples obtained from a patient (e.g., a human patient). In preferred embodiments, a sample is obtained from tissue, bodily fluid, or microorganisms collected from a subject. Sample sources include, but are not limited to, mucus, sputum (processed or unprocessed), bronchial alveolar lavage (BAL), bronchial wash (BW), blood (e.g., whole blood, plasma, and serum), bodily fluids, cerebrospinal fluid (CSF), urine, plasma, serum, or tissue (e.g., biopsy material). Preferred sample sources include nasopharyngeal swabs and BAL.

As used herein, "Scorpion primer" refers to an oligonucleotide comprising a 3' primer with a 5' extended probe tail comprising a hairpin structure which possesses a fluorophore/quencher pair. Optionally, the Scorpion primer further contains an amplification blocker (e.g., hexethylene glycol ("HEG") separating the probe moiety from the primer moiety. As described in more detail herein, the Scorpion™ primers are examples of Scorpion primers.

As used herein, the term "Scorpion™ detection system" refers to a method for real-time PCR. This method utilizes a bi-functional molecule (referred to herein as a "Scorpion™"), which contains a PCR primer element covalently linked by a polymerase-blocking group to a probe element. Additionally, each Scorpion™ molecule contains a fluorophore that interacts with a quencher to reduce the background fluorescence.

The terms "target nucleic acid" or "target sequence" as used herein refer to a sequence which includes a segment of nucleotides of interest to be amplified and detected. Copies of the target sequence which are generated during the amplification reaction are referred to as amplification products, amplimers, or amplicons. Target nucleic acid may be composed of segments of a chromosome, a complete gene with or without intergenic sequence, segments or portions of a gene with or without intergenic sequence, or sequence of nucleic acids which probes or primers are designed. Target nucleic acids may include a wild-type sequence(s), a mutation, deletion or duplication, tandem repeat regions, a gene of interest, a region of a gene of interest or any upstream or downstream region thereof. Target nucleic acids may represent alternative sequences or alleles of a particular gene. Target nucleic acids may be derived from genomic DNA, cDNA, or RNA. As used herein target nucleic acid may be DNA or RNA extracted from a cell or a nucleic acid copied or amplified therefrom, or may include extracted nucleic acids further converted using a bisulfite reaction.

As used herein "TaqMan® PCR detection system" refers to a method for real time PCR. In this method, a TaqMan® probe which hybridizes to the nucleic acid segment amplified is included in the PCR reaction mix. The TaqMan® probe comprises a donor and a quencher fluorophore on either end of the probe and in close enough proximity to each other so that the fluorescence of the donor is taken up by the quencher. However, when the probe hybridizes to the amplified segment, the 5'-exonuclease activity of the Taq polymerase cleaves the probe thereby allowing the donor fluorophore to emit fluorescence which can be detected.

### Biological Sample Collection and Preparation

The methods and compositions of this invention may be used to detect pathogens that cause atypical pneumonia by detecting pathogen nucleic acids in a biological sample obtained from an individual. Samples for pathogen detection may also comprise cultures of isolated bacteria grown on appropriate media to form colonies, wherein the cultures were prepared from a biological sample obtained from an individual.

The nucleic acid (DNA or RNA) may be isolated from the sample according to any methods well known to those of skill in the art. If necessary the sample may be collected or concentrated by centrifugation and the like. The cells of the sample may be subjected to lysis, such as by treatments with enzymes, heat, surfactants, ultrasonication, or a combination thereof. The lysis treatment is performed in order to obtain a sufficient amount of DNA derived from the pathogens, if present in the sample, to detect using polymerase chain reaction.

Various methods of DNA extraction are suitable for isolating the DNA. Suitable methods include phenol and chloroform extraction. See Maniatis et al., Molecular Cloning, A Laboratory Manual, 2d, Cold Spring Harbor Laboratory Press, page 16.54 (1989). Numerous commercial kits also yield suitable DNA including, but not limited to, QIAamp™ mini blood kit, Agencourt Genfind™, Roche Cobas® Roche MagNA Pure® or phenol: chloroform extraction using Eppendorf Phase Lock Gels®.

### Target Nucleic Acids and Primers

In various embodiments of the present invention, oligonucleotide primers and probes are used in the methods described herein to amplify and detect target sequences of atypical pneumonia-inducing pathogens. In certain embodiments, target nucleic acids may include the P1 gene or gene fragments from *M. pneumoniae,* the Cpn0980 gene or gene fragments from *C. pneumoniae,* and the pmiA gene or gene fragments from *L. pneumophila.* In addition, primers can also be used to amplify one or more control nucleic acid sequences. The target nucleic acids described herein may be detected singly or in a multiplex format, utilizing individual labels for each target.

The skilled artisan is capable of designing and preparing primers that are appropriate for amplifying a target sequence in view of this disclosure. The length of the amplification primers for use in the present invention depends on several factors including the nucleotide sequence identity and the temperature at which these nucleic acids are hybridized or used during in vitro nucleic acid amplification. The considerations necessary to determine a preferred length for an amplification primer of a particular sequence identity are well known to the person of ordinary skill in the art.

Primers that amplify a nucleic acid molecule can be designed using, for example, a computer program such as OLIGO (Molecular Biology Insights, Inc., Cascade, CO). Important features when designing oligonucleotides to be used as amplification primers include, but are not limited to, an appropriate size amplification product to facilitate detection (e.g., by electrophoresis or real-time PCR), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 15 to 35 nucleotides in length.

Designing oligonucleotides to be used as hybridization probes can be performed in a manner similar to the design of primers. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are generally 15 to 60 nucleotides in length.

In some embodiments, a mix of primers is provided having degeneracy at one or more nucleotide positions. Degenerate primers are used in PCR where variability exists in the target sequence, i.e. the sequence information is ambiguous. Typically, degenerate primers will exhibit variability at no more than about 4, no more than about 3, preferably no more than about 2, and most preferably, no more than about 1 nucleotide position.

In a suitable embodiment, PCR is performed using a bifunctional primer/probe combination (e.g., Scorpion™ primers). Scorpion primers, as used in the present invention comprise a 3' primer with a 5' extended probe tail comprising a hairpin structure which possesses a fluorophore/quencher pair. During PCR, the polymerase is blocked from extending into the probe tail by the inclusion of hexethlyene glycol (HEG). The hairpin structure is formed by two self-complementary sections of single stranded DNA which anneal (hybridize), and form a single-stranded (non-complementary) loop region containing some or all of the probe sequence. During the first round of amplification the 3' target-specific primer anneals to the target and is extended such that the Scorpion™ is now incorporated into the newly synthesized strand, which possesses a newly synthesized target region for the 5' probe. During the next round of denaturation and annealing, the probe region of the Scorpion primer hairpin loop will hybridize to the target, thus separating the fluorophore and quencher and creating a measurable signal. Such probes are described in Whitcombe et al., Nature Biotech 17: 804-807 (1999).

### Amplification of Nucleic Acids

Nucleic acid samples or isolated nucleic acids may be amplified by various methods known to the skilled artisan. Preferably, PCR is used to amplify nucleic acids of interest. Briefly, in PCR, two primer sequences are prepared that are complementary to regions on opposite complementary strands of the marker sequence. An excess of deoxynucleotide triphosphates are added to a reaction mixture along with a DNA polymerase, e.g., Taq polymerase. When the template is sequence-modified, as described above, the amplification mixture preferably does not contain a UNG nuclease.

If the target sequence is present in a sample, the primers will bind to the sequence and the polymerase will cause the primers to be extended along the target sequence by adding on nucleotides. By raising and lowering the temperature of the reaction mixture, the extended primers will dissociate from the marker to form reaction products, excess primers will bind to the marker and to the reaction products and the process is repeated, thereby generating amplification products. Cycling parameters can be varied, depending on the length of the amplification products to be extended. An internal positive amplification control (IPC) can be included in the sample, utilizing oligonucleotide primers and/or probes. The IPC can be used to monitor both the conversion process and any subsequent amplification.

In a suitable embodiment, real time PCR is performed using any suitable instrument capable of detecting the accumulation of the PCR amplification product. Most commonly, the instrument is capable of detecting fluorescence from one or more fluorescent labels. For example, real time detection on the instrument (e.g. a ABI Prism® 7900HT sequence detector) monitors fluorescence and calculates the measure of reporter signal, or Rn value, during each PCR cycle. The threshold cycle, or Ct value, is the cycle at which fluorescence intersects the threshold value. The threshold value is determined by the sequence detection system software or manually.

### Detection of Amplified Target Nucleic Acids

Amplification of nucleic acids can be detected by any of a number of methods well-known in the art such as gel electrophoresis, column chromatography, hybridization with a probe, sequencing, melting curve analysis, or "real-time" detection.

In the preferred approach, sequences from two or more fragments of interest are amplified in the same reaction vessel (i.e. "multiplex PCR"). Detection can take place by measuring the end-point of the reaction or in "real time." For real-time detection, primers and/or probes may be detectably labeled to allow differences in fluorescence when the primers become incorporated or when the probes are hybridized, for example, and amplified in an instrument capable of monitoring the change in fluorescence during the reaction. Real-time detection methods for nucleic acid amplification are well known and include, for example, the TaqMan® system, Scorpion™ primer system and use of intercalating dyes for double stranded nucleic acid.

In end-point detection, the amplicon(s) could be detected by first size-separating the amplicons, then detecting the size-separated amplicons. The separation of amplicons of different sizes can be accomplished by, for example, gel electrophoresis, column chromatography, or capillary electrophoresis. These and other separation methods are well-known in the art. In one example, amplicons of about 10 to about 150 base pairs whose sizes differ by 10 or more base pairs can be separated, for example, on a 4% to 5% agarose gel (a 2% to 3% agarose gel for about 150 to about 300 base pair amplicons), or a 6% to 10% polyacrylamide gel. The separated nucleic acids can then be stained with a dye such as ethidium bromide and the size of the resulting stained band or bands can be compared to a standard DNA ladder.

In some embodiments, amplified nucleic acids are detected by hybridization with a specific probe. Probe oligonucleotides, complementary to a portion of the amplified target sequence may be used to detect amplified fragments. Hybridization may be detected in real time or in non-real time. Amplified nucleic acids for each of the target sequences may be detected simultaneously (i.e., in the same reaction vessel) or individually (i.e., in separate reaction vessels). In preferred embodiments, the amplified DNA is detected simultaneously, using two or more distinguishably-labeled, gene-specific oligonucleotide probes, one which hybridizes to the first target sequence and one which hybridizes to the second target sequence. For sequence-modified nucleic acids, the target may be independently selected from the top strand or the bottom strand. Thus, all targets to be detected may comprise top strand, bottom strand, or a combination of top strand and bottom strand targets.

The probe may be detectably labeled by methods known in the art. Useful labels include, for example, fluorescent dyes (e.g., Cy5®, Cy3®, FITC, rhodamine, lanthamide phosphors, Texas red, FAM, JOE, Cal Fluor Red 610®, Quasar 670®), radioisotopes (e.g., ³²P, ³⁵S, ³H, ¹⁴C, ¹²⁵I, ¹³¹I), electron-dense reagents (e.g., gold), enzymes (e.g., horseradish peroxidase, beta-galactosidase, luciferase, alkaline phosphatase), colorimetric labels (e.g., colloidal gold), magnetic labels (e.g., Dynabeads™), biotin, dioxigenin, or haptens and proteins for which antisera or monoclonal antibodies are available. Other labels include ligands or oligonucleotides capable of forming a complex with the corresponding receptor or oligonucleotide complement, respectively. The label can be directly incorporated into the nucleic acid to be detected, or it can be attached to a probe (e.g., an oligonucleotide) or antibody that hybridizes or binds to the nucleic acid to be detected.

One general method for real time PCR uses fluorescent probes such as the TaqMan® probes, molecular beacons, and Scorpions. Real-time PCR quantifies the initial amount of the template with more specificity, sensitivity and reproducibility, than other forms of quantitative PCR, which detect the amount of final amplified product. Real-time PCR does not detect the size of the amplicon. The probes employed in Scorpion™ and TaqMan® technologies are based on the principle of fluorescence quenching and involve a donor fluorophore and a quenching moiety.

In a preferred embodiment, the detectable label is a fluorophore. The term "fluorophore" as used herein refers to a molecule that absorbs light at a particular wavelength (excitation frequency) and subsequently emits light of a longer wavelength (emission frequency). The term "donor fluorophore" as used herein means a fluorophore that, when in close proximity to a quencher moiety, donates or transfers emission energy to the quencher. As a result of donating energy to the quencher moiety, the donor fluorophore will itself emit less light at a particular emission frequency that it would have in the absence of a closely positioned quencher moiety.

The term "quencher moiety" as used herein means a molecule that, in close proximity to a donor fluorophore, takes up emission energy generated by the donor and either dissipates the energy as heat or emits light of a longer wavelength than the emission wavelength of the donor. In the latter case, the quencher is considered to be an acceptor fluorophore. The quenching moiety can act via proximal (i.e., collisional) quenching or by Förster or fluorescence resonance energy transfer ("FRET"). Quenching by FRET is generally used in TaqMan® probes while proximal quenching is used in molecular beacon and Scorpion™ type probes.

Suitable fluorescent moieties include the following fluorophores known in the art: 4-acetamido-4'-isothiocyanatostilbene-2,2'disulfonic acid, acridine and derivatives (acridine, acridine isothiocyanate) Alexa Fluor® 350, Alexa Fluor® 488, Alexa Fluor® 546, Alexa Fluor® 555, Alexa Fluor® 568, Alexa Fluor® 594, Alexa Fluor® 647 (Molecular Probes), 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, BODIPY® R-6G, BOPIPY® 530/550, BODIPY® FL, Brilliant Yellow , coumarin and derivatives (coumarin, 7-amino-4-methylcoumarin (AMC, Coumarin 120), 7-amino-4-trifluoromethylcouluarin (Coumarin 151)), Cy2®, Cy3®, Cy3.5®, Cy5®, Cy5.5®, cyanosine, 4',6-diaminidino-2-phenylindole (DAPI), 5', 5"-dibromopyrogallol-sulfonephthalein (Bromopyrogallol Red), 7-diethylamino-3-(4'-isothiocyanatophenyl)-4-methylcoumarin, diethylenetriamine pentaacetate, 4,4'-diisothiocyanatodihydro-stilbene-2,2'-disulfonic acid, 4,4'-diisothiocyanatostilbene-2,2'-disulfonic acid, 5-[dimethylamino]naphthalene-1-sulfonyl chloride (DNS, dansyl chloride), 4-(4'-dimethylaminophenylazo)benzoic acid (DABCYL), 4-dimethylaminophenylazophenyl-4'-isothiocyanate (DABITC), EclipseTM (Epoch Biosciences Inc.), eosin and derivatives (eosin, eosin isothiocyanate), erythrosin and derivatives (erythrosin B, erythrosin isothiocyanate), ethidium, fluorescein and derivatives (5-carboxyfluorescein (FAM), 5-(4,6-dichlorotriazin-2-yl)aminofluorescein (DTAF), 2',7'-dimethoxy-4'5'-dichloro-6-carboxyfluorescein (JOE), fluorescein, fluorescein isothiocyanate (FITC), hexachloro-6-carboxyfluorescein (HEX), QFITC (XRITC), tetrachlorofluorescein (TET)), fluorescamine, IR144, IR1446, Malachite Green isothiocyanate, 4-methylumbelliferone, ortho cresolphthalein, nitrotyrosine, pararosaniline, Phenol Red, B-phycoerythrin, R-phycoerythrin, o-phthaldialdehyde, Oregon Green®, propidium iodide, pyrene and derivatives (pyrene, pyrene butyrate, succinimidyl 1-pyrene butyrate), QSY® 7, QSY® 9, QSY® 21, QSY® 35 (Molecular Probes), Reactive Red 4 (Cibacron® Brilliant Red 3B-A), rhodamine and derivatives (6-carboxy-X-rhodamine (ROX), 6-carboxyrhodamine (R6G), lissamine rhodamine B sulfonyl chloride, rhodamine (Rhod), rhodamine B, rhodamine 123, rhodamine green, rhodamine X isothiocyanate, sulforhodamine B, sulforhodamine 101, sulfonyl chloride derivative of sulforhodamine 101 (Texas Red)), N,N,N',N'-tetramethyl-6-carboxyrhodamine (TAMRA), tetramethyl rhodamine, tetramethyl rhodamine isothiocyanate (TRITC), riboflavin, rosolic acid, terbium chelate derivatives.

Other fluorescent nucleotide analogs can be used, see, e.g., Jameson, 278 Meth. Enzymol. 363-390 (1997); Zhu, 22 Nucl. Acids Res. 3418-3422 (1994). U.S. Patent Nos. 5,652,099 and 6,268,132 also describe nucleoside analogs for incorporation into nucleic acids, e.g., DNA and/or RNA, or oligonucleotides, via either enzymatic or chemical synthesis to produce fluorescent oligonucleotides. U.S. Patent No. 5,135,717 describes phthalocyanine and tetrabenztriazaporphyrin reagents for use as fluorescent labels.

Suitable quenchers are selected based on the fluorescence spectrum of the particular fluorophore. Useful quenchers include, for example, the Black Hole™ quenchers BHQ-1, BHQ-2, and BHQ-3 (Biosearch Technologies, Inc.), and the ATTO-series of quenchers (ATTO 540Q, ATTO 580Q, and ATTO 612Q; Atto-Tec GmbH).

The detectable label can be incorporated into, associated with or conjugated to a nucleic acid. Label can be attached by spacer arms of various lengths to reduce potential steric hindrance or impact on other useful or desired properties. See, e.g., Mansfield, 9 Mol. Cell. Probes 145-156 (1995). Detectable labels can be incorporated into nucleic acids by covalent or non-covalent means, e.g., by transcription, such as by random-primer labeling using Klenow polymerase, or nick translation, or amplification, or equivalent as is known in the art. For example, a nucleotide base is conjugated to a detectable moiety, such as a fluorescent dye, and then incorporated into nucleic acids during nucleic acid synthesis or amplification.

With Scorpion primers, sequence-specific priming and PCR product detection is achieved using a single molecule. The Scorpion primer maintains a stem-loop configuration in the unhybridized state. The fluorophore is attached to the 5' end and is quenched by a moiety coupled to the 3' end, although in suitable embodiments, this arrangement may be switched. The 3' portion of the stem also contains sequence that is complementary to the extension product of the primer. This sequence is linked to the 5' end of a specific primer via a non-amplifiable monomer. After extension of the primer moiety, the specific probe sequence is able to bind to its complement within the extended amplicon thus opening up the hairpin loop. This prevents the fluorescence from being quenched and a signal is observed. A specific target is amplified by the reverse primer and the primer portion of the Scorpion primer, resulting in an extension product. A fluorescent signal is generated due to the separation of the fluorophore from the quencher resulting from the binding of the probe element of the Scorpion primer to the extension product.

TaqMan® probes (Heid, et al., Genome Res 6: 986-994, 1996) use the fluorogenic 5' exonuclease activity of Taq polymerase to measure the amount of target sequences in cDNA samples. TaqMan® probes are oligonucleotides that contain a donor fluorophore usually at or near the 5' base, and a quenching moiety typically at or near the 3' base. The quencher moiety may be a dye such as TAMRA or may be a non-fluorescent molecule such as 4-(4 -dimethylaminophenylazo) benzoic acid (DABCYL). See Tyagi, et al., 16 Nature Biotechnology 49-53 (1998). When irradiated, the excited fluorescent donor transfers energy to the nearby quenching moiety by FRET rather than fluorescing. Thus, the close proximity of the donor and quencher prevents emission of donor fluorescence while the probe is intact.

TaqMan® probes are designed to anneal to an internal region of a PCR product. When the polymerase (e.g., reverse transcriptase) replicates a template on which a TaqMan® probe is bound, its 5' exonuclease activity cleaves the probe. This ends the activity of the quencher (no FRET) and the donor fluorophore starts to emit fluorescence which increases in each cycle proportional to the rate of probe cleavage. Accumulation of PCR product is detected by monitoring the increase in fluorescence of the reporter dye (note that primers are not labeled). If the quencher is an acceptor fluorophore, then accumulation of PCR product can be detected by monitoring the decrease in fluorescence of the acceptor fluorophore.

### Detection of Mycoplasma pneumoniae

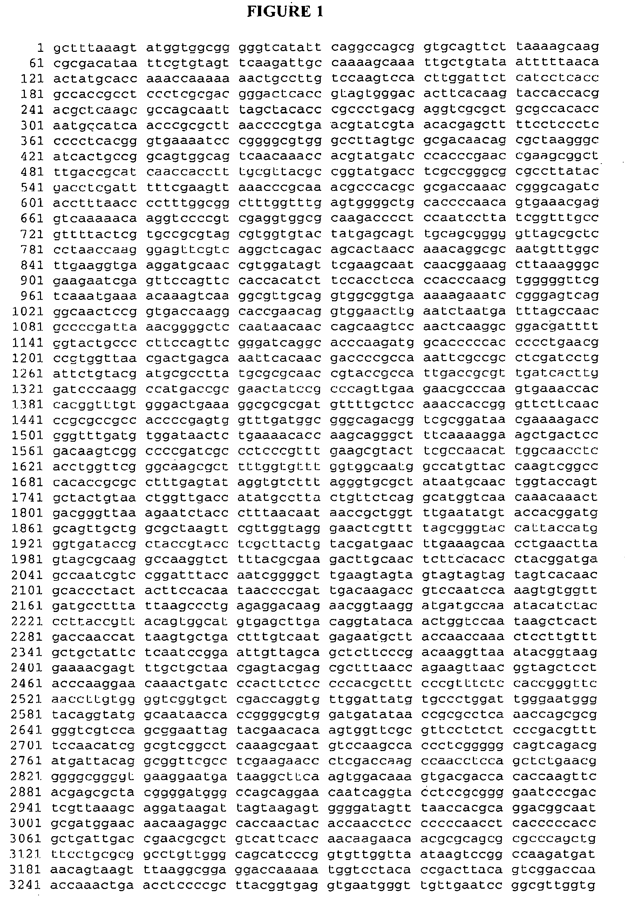

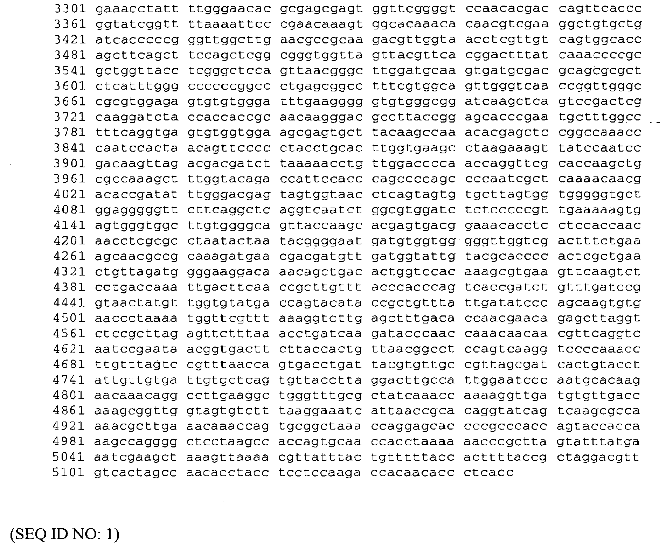

*M. pneumoniae* is one organism that causes atypical pneumonia. The presence of *M. pneumoniae* in a patient may be determined by detecting an *M. pneumoniae* target nucleic acid in a biological sample. Suitable target nucleic acids include, for example, the *M*. *pneumoniae* P1 (cytoadhesin) gene and fragments thereof. The nucleotide sequence of the P1 gene is provided at Genbank Accession No. AF290002 (Dorigo-Zetsma, et al., Infect. Immun. 69: 5612-5618, 2001) and is shown in FIGURE 1 (SEQ ID NO: 1).

In preferred embodiments, the target nucleic acid corresponds to nucleotides 2601-2800 of the P1 gene or a fragment thereof, and is provided below as SEQ ID NO: 2.

1 ccggggcgtg gatgatataa ccgcgcctca aaccagcgcg gggtcgtcca
51 gcggaattag tacgaacaca agtGGTTCGC GTTCCTCTCT CCCgacgttt
101 tccaacatcg gcgtcggcct caaagcgaat gtccaagcca ccctcggggg
151 cagtcagacg atgattacag gcggttcgcc tcgaagaacc ctcgaccaag
SEQ ID NO: 2

This target sequence of SEQ ID NO: 2 shows a poor sequence alignment with the *M. genitalium* homolog (Dallo et al., Infect. Immun. 57: 1059-1065, 1989). The full target sequence, or any portion thereof, may be amplified and detected using any appropriate primers and probes. Particularly useful primers and probes include, for example, those directed to nucleotides 2649-2671 (SEQ ID NO: 4) and 2726-2743 (SEQ ID NO: 5) of the P1 gene, or complements thereof (underlined in the target nucleic acid provided above). One useful probe (capitalized in the target nucleic acid provided above) is directed to nucleotides 2674-2693 (SEQ ID NO: 3) of the P1 gene, or a complement thereof.

### Detection of Chlamydophila pneumoniae

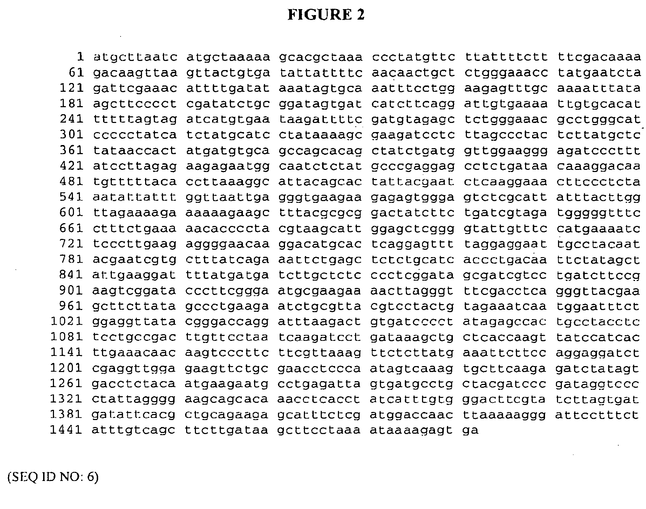

*C. pneumoniae* is another organism that causes atypical pneumonia. The presence of C. *pneumoniae* in a patient may be determined by detecting a C. *pneumoniae* target nucleic acid in the biological sample. Suitable nucleic acids include, for example, the Cpn0980 gene and fragments thereof. The sequence of the Cpn0980 gene from *C. pneumonia* CWL029 is shown in FIGURE 2 (SEQ ID NO: 6).

In preferred embodiments, the target nucleic acid corresponds to nucleotides 501-750 of the Cpn0980 gene or a fragment thereof, and is provided below as SEQ ID NO: 7.

1 attacagcac tattacgaat ctcaaggaaa cttccctcta aatattattt

51 ggttaattga gggtgaagaa gagagtggga gtctcgcatt atttacttgg

101 ttagaaaaga aaaaagaagc tttacgCGCG GACTATCTTC TGATCGTaga

151 tgggggtttc ctttctgaaa aacaccccta cgtaagcatt ggagctcggg

201 gtattgtttc catgaaaatc tcccttgaag aggggaacaa ggacatgcac

SEQ ID NO: 7

This target sequence has no apparent mammalian homology and is absent in *C*. *trachomatis* (Kalman et al., Nat. Genet. 99: 385-389, 1999; Table 2) and *C*. *psittaci* by a comparative genomic study. The full target sequence, or any portion thereof, may be amplified and detected using any appropriate primers and probes. Particularly useful primers include, for example, primers directed to nucleotides 578-602 (SEQ ID NO: 9) and 683-700 (SEQ ID NO: 10) of the Cpn0980 gene, or complements thereof (underlined in the target nucleic acid provided above). One useful probe (capitalized in the target nucleic acid provided above) are directed to nucleotides 627-647 (SEQ ID NO: 8) of the Cpn0980 gene, or a complement thereof.

### Detection of Legionella pneumophila

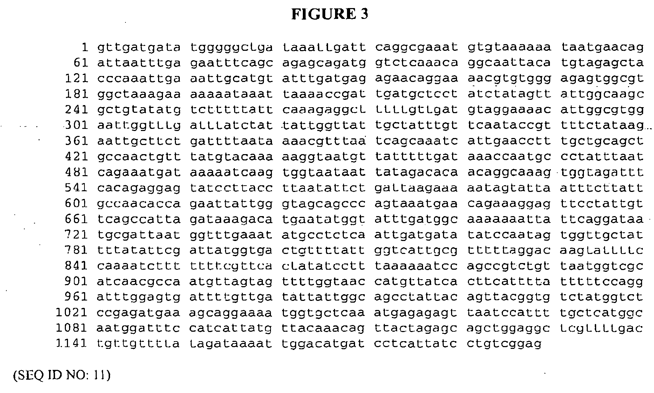

*L. pneumophila* is another organism that causes atypical pneumonia. The presence *of L. pneumophila* in a patient may be determined by detecting an *L. pneumophila* target nucleic acid in the biological sample. Suitable nucleic acids include, for example, the genes that encode the pmiA gene and fragments thereof. The nucleotide sequence of the pmiA gene is provided at Genbank Accession No. AB193439 (Miyake, et al., Infect. Immun. 73: 6272-6282, 2005) and is shown in FIGURE 3 (SEQ ID NO: 11).

In preferred embodiments, the target nucleic acid corresponds to nucleotides 91-260 of the pmiA gene or a fragment thereof, and is provided below as SEQ ID NO: 12.

1 gtctcaaaca ggcaattaca tgtagagcta cccaaattga aattgcatgt atttgatgag

61 agaacaggaa aacgtGTGGG AGAGTGGCGT GGCtaaagaa aaaaataaat taaaaccgat

121 tgatgctcct atctatagtt attggcaagc gctgtatatg tctttttatt

SEQ ID NO: 12

This target sequence of SEQ ID NO: 12 is present and relatively conserved in serogroups 1, 3, 4, 5, 6, and 8 *of L. pneumophila,* but not in other *Legionella* species. It is believed that the pmiA gene is involved in infectivity of *L. pneumophila* and is therefore likely conserved among different serogroups, including those for which no (or limited) genomic sequence information is available.

Although any suitable region within the pmiA target sequence may be amplified and detected as an indictor of the presence *of L. pneumophila,* particularly useful primers and probes include, for example, those directed to nucleotides 135-159 (SEQ ID NO: 14) and nucleotides 211-252 of the pmiA gene (underlined in the target sequence provided above) or complements thereof. Specific primers and probes directed to the latter region include those directed to nucleotides 211-237 (SEQ ID NO: 15), 213-239 (SEQ ID NO: 16), and 232-252 (SEQ ID NO: 17) of the pmiA gene (corresponding to nucleotides 121-147, 123-149, and 232-252 of SEQ ID NO: 12, respectively), or complements thereof. One useful probe (capitalized in the target nucleic acid provided above) may be directed to nucleotides 166-183 of the pmiA gene (SEQ ID NO: 13), or complements thereof.

### EXAMPLES

### EXAMPLE 1: Sample Collection and DNA Extraction.

A total of 320 samples were prepared and tested for assay validation purposes. The samples were either actual clinical specimens obtained from patients or contrived samples which were prepared from pathogen-negative biological material and spiked with the indicated pathogenic bacteria. Of the 320 samples, 188 samples were negative and consisted of 164 swabs, 20 BAL, and 4 BW. The positive samples were as follows:

| | Clinical | Contrived | | |
|---|---|---|---|---|
| | All | Swab | BAL | Total |
| *M. pneumoniae* | 30 | 6 | 6 | 42 |
| *C. pneumoniae* | 2 | 27 | 12 | 41 |
| *L. pneumophila* | 7 | 25 | 11 | 43 |
| Dual Positives | 0 | 0 | 6 | 6 |
| | 39 | 58 | 35 | 132 |

BAL and BW samples were frozen, undiluted, at -20°C for transport, and stored at -70°C until assay. Swabs were placed immediately into Micro Test™ M4 media (Remel, Inc.), frozen at -20°C for transport, and stored at -70°C until assay.

DNA was extracted from a 250 µl aliquot of the BAL and nasopharyngeal swab samples. Briefly, the samples were thawed, aliquoted into 1.5 ml Eppendorf tubes. Additional tubes containing 250 µl of diluent with positive control DNA (see below) or no DNA (negative control) were prepared and processed simultaneously with the patient samples. All samples (including controls) were centrifuged for 10 min at 13,000 rpm (2-8°C). Next, 150 µl of the supernatant was carefully aspirated and discarded, leaving about 100 µl of sample in the tube. The pellet was resuspended by adding 100 µl of ATL Buffer ATL and 20 µl of Proteinase K solution to the remaining sample, followed by gentle vortexing. Samples were incubated at about 55°C for 30 min with occasional vortexing, followed by the addition of 200 µl of Buffer AL. To each sample was next added 5 µl of Internal Control DNA (see below). The QIAamp™ mini blood kit (Qiagen) was used to extract the DNA according to the manufacturer's instructions.

### Internal Control

The Internal Control (IC) DNA consisted of a DNA fragment of random sequence not present in any of the assayed organisms. The IC was created by annealing and cloning two 5'-phosphate-labeled oligonucleotides (pIC-F-5'P and pIC-R-5'P; SEQ ID NOs: 26 and 27, respectively) into the EcoRI site of pUC19. The IC sequence was amplified using the forward and reverse primers of SEQ ID NOs: 28 and 29, respectively. This resulted in an IC the nucleic acid of SEQ ID NO: 30. The specific oligonucleotides used to create the IC are as follows:
pIC-F-5'P: 
aattcgccct ttgtttcgac ctagcttgcc agttcgcaga atttgttgct
cgtcagtcgt cggcggtttt aagggcg (SEQ ID NO: 26);
pIC-R-5'P: 
aattcgccct taaaaccgcc gacgactgac gagcaacaaa ttctgcgaac
tggcaagcta ggtcgaaaca aagggcg (SEQ ID NO: 27)
Forward Primer: gttttcccagtcacgacgttgta (SEQ ID NO: 28)
Reverse Primer: cactttatgcttccggctcgta (SEQ ID NO: 29)
Internal Control:

gttttcccag tcacgacgtt gtaaaacgac ggccagtgaa ttcgccctta
aaaccgccga cgactgacga gcaacaaatt ctgcgaactg gcaagctagg tcgaaacaaa
gggcggattc gagctcggta cccggggatc ctctagagtc gacctgcagg catgcaagct
tggcgtaatc atggtcatag ctgtttcctg tgtgaaattg ttatccgctc acaattccac
acaacatacg agccggaagc ataaagtg (SEQ ID NO: 30)

### Positive and Negative Controls

For all assays, positive and negative controls were prepared and run simultaneously with the patient samples. Negative control assays contained no DNA.

The positive control DNA consists of a DNA fragment containing target regions of *M. pneumoniae, C. pneumoniae,* and *L. pneumophila* in a single molecule. The positive control DNA was created as follows. Target sequences from M. pneumoniae (ATCC 15531), C. pneumoniae (ATCC VR-1360), and L. pneumophila (ATCC 33152) were independently amplified using the following primers:
Mpn2-F1 EcoRI: accagggaattcagcggaattagtacgaacacaa (SEQ ID NO: 31)
Mpn2-R1 BamHI: tgacttggatccgggtggcttggacattcg (SEQ ID NO: 32)
Cpn2-F1 BamHI: tgaagaggatccggagtctcgcattatttacttggt (SEQ ID NO: 33)
Cpn2-R2 PstI: gaaacactgcagcccgagctccaatgctta (SEQ ID NO: 34)
Lpn3-F3 PstI: caaatctgcaggcatgtatttgatgagagaacagga (SEQ ID NO: 35)
Lpn3-R2 HindIII: cagataagcttgacatatacagcgcttgccaa (SEQ ID NO: 36)

Each amplicon was purified and treated with the indicated restriction enzymes and cloned into a pUC19 vector treated with EcoRI and HindIII. The positive control amplicon was generated using the primers of SEQ ID NOs: 37-38 to yield a nucleic acid having the sequence of SEQ ID NO: 39.
Forward Primer: gttttcccag tcacgacgtt gta (SEQ ID NO: 37)
Reverse Primer: cactttatgc ttccggctcg ta (SEQ ID NO: 38)
Positive Control:

gttttcccag tcacgacgtt gtaaaacgac ggccagtgaa ttcagcggaa ttagtacgaa
cacaagtggt tcgcgttcct ctctcccgac gttttccaac atcggcgtcg gcctcaaagc
gaatgtccaa gccacccgga tccggagtct cgcattattt acttggttag aaaagaaaaa
agaagcttta cgcgcggact atcttctgat cgtagatggg ggtttccttt ctgaaaaaca
cccctacgta agcattggag ctcgggctgc aggcatgtat ttgatgagag aacaggaaaa
cgtgtgggag agtggcgtgg ctaaagaaaa aaataaatta aaaccgattg atgctcctat
ctatagttat tggcaagcgc tgtatatgtc aagcttggcg taatcatggt catagctgtt
tcctgtgtga aattgttatc cgctcacaat tccacacaac atacgagccg gaagcataaa
gtg (SEQ ID NO: 39)

### EXAMPLE 2: Multiplex PCR Detection of Atypical Pneumonia Pathogens.

The PCR Reagent Solution contained the following: Tris-HCl (pH 9.0), MgCl₂, KCl, EDTA, DTT, Tween 20, (NH₄)₂SO₄, glycerol, dNTPs (dT, dA, dG, and dC), and FastStart DNA Polymerase™.

A PCR Primer Solution was prepared containing 200 nM of each of the Scorpion primers and reverse primers for *M. pneumoniae, C. pneumoniae, L. pneumophila,* and 100 nM of the internal control primers. The primer pairs were as follows:

*M. pneumoniae:*
[Q]-agcGGGAGAGAGGAACGCGAACCcgct-[F]-heg-cagcggaattagtacgaacacaa (SEQ ID NOs: 18 and 45, respectively), and
Reverse Primer: gggtggcttggacattcg (SEQ ID NO: 19).

*C. pneumoniae:*
[F]-ccgACGATCAGAAGATAGTCCGCGcgtcgg-[Q]-heg-ggagtctcgcattatttacttggt (SEQ ID NOs: 20 and 46, respectively), and
Reverse Primer: cccgagctccaatgctta (SEQ ID NO: 21).

*L. pneumophila* was assayed using one of two different primer pairs. The primer pairs were:

Primer pair #1:
[Q]-agcgccGTGGGAGAGTGGCGTGGCgct-[F]-heg-tgccaataactatagataggagcatca (SEQ ID NOs: 22 and 47, respectively), and
Reverse Primer: gcatgtatttgatsagagaacagga (SEQ ID NO: 23); or

The "S" in the reverse primer indicates that degenerate reverse primers were used, wherein the "S" is either cytosine or guanine. It is recognized that either member of the degenerate reverse primers of SEQ ID NO: 23 may be used individually.

Primer pair #2:
[Q]-agcGCCACGCCACTCTCCCACggcgct-[F]-heg-gcatgtatttgatgagagaacagga (SEQ ID NOs: 24 and 48, respectively), and
Reverse Primer: cttgccaataactatagataggagcat (SEQ ID NO: 25).

Internal Contol:
(Q)-agcgtgcgaactggcaagcacgct(F)-heg-attcgccctttgtttcgaccta (SEQ ID NOs: 40 and 49, respectively), and
Reverse Primer: ccgacgactgacgagcaa (SEQ ID NO: 41).

For each Scorpion primer listed above, Q = quencher, F = fluorophore, and "heg" = hexethylene glycol. The probe sequence is identified by capital letters.

| Scorpion Primer | F | Excitation | Emission | Target Gene |
|---|---|---|---|---|
| M. pneumoniae | FAM | 495 nm | 520 nm | P1 |
| C. pneumoniae | JOE | 520 nm | 548 nm | Cpn0980 |
| L. pneumophila | CalFlour Red 610 | 583 nm | 603 nm | pmiA |
| Internal Control | Quasar 670 | 649 nm | 670 nm | See above |

The PCR Reaction Solution was created by mixing 12.5 µl of PCR Reagent Solution, 2.5 µl PCR Primer Solution, and 5 µl of nuclease-free water per sample assay to be performed.

Multiplex PCR detection assays were performed in 96-well plates. Each assay contained 20 µl of the PCR Reaction Solution and 5 µl of extracted nucleic acid (i.e., patient sample, positive control, or negative control). The plates were sealed, centrifuged at 2000 xg for 2 minutes, and run in an Applied Biosystems (ABI) 7500 Real-Time PCR Detection System. The PCR reaction was performed as follows:

| | |
|---|---|
| Stage 1 (one cycle): | 10 minutes at 95°C |
| Stage 2 (45 cycles): | Step 1: 95°C for 15 sec |
| | Step 2: 60°C for 35 sec (data collection during Step 2). |

The data was collected and patient samples analyzed for the presence of one or more pathogenic species. Patient samples having a positive result for any one or more of the pathogenic species were scored as positive for those species. Patient samples having a negative result for all pathogenic species were only scored as negative provided that the internal control target nucleic acid was detected and the positive control sample assay was positive for all three target nucleic acids.

Of the initial 320 samples, valid assays were obtained from 311 and are reported herein.

### EXAMPLE 3: Confirmation of Multiplex PCR Assay Results.

The results of the multiplex assay described above were compared to other detection assays. Aliquots of all samples were run in a secondary assay for confirmation of the multiplex assay result. *M. pneumoniae* and *C*. *pneumoniae* were assessed in the ProPneumo-1 assay (Prodesse, Inc.). This assay simultaneously detects the 16S-23S rRNA gene of *M. pneumoniae* and the OmpA gene of *C*. *pneumoniae* using real-type PCR with fluorescence detection.

*L. pneumophila* was assessed using a Taqman®-style assay for a single gene. Briefly, the target sequence was nucleotides 14-121 of the *mip* gene which was assessed using the following primers and probe:
Forward primer: tggtgactgcagctgttatgg (SEQ ID NO: 42)
Reverse primer: cggcaccaatgctataagacaa (SEQ ID NO: 43)
Probe: atggctgcaaccgatgccacatc (SEQ ID NO: 44)

The results for the 311 samples that yielded valid results in the multiplex assay described above are as follows:
*M. pneumoniae:*

| | | Multiplex Assay Results | | |
|---|---|---|---|---|
| | | Positive | Negative | Total |
| ProPneumo-1 Assay Results | Positive | 43 | 1 | 44 |
| | Negative | 2 | 265 | 267 |
| | Total | 45 | 266 | 311 |

| | | | | |
|---|---|---|---|---|
| Sensitivity = 97.7%; Specificity = 99.3%; Concordance = 99.0% | | | | |

*C. pneumoniae:*

| | | Multiplex Assay Results | | |
|---|---|---|---|---|
| | | Positive | Negative | Total |
| ProPneumo-1 Assay Results | Positive | 40 | 1 | 41 |
| | Negative | 1 | 269 | 270 |
| | Total | 41 | 270 | 311 |

| | | | | |
|---|---|---|---|---|
| Sensitivity = 97.6%; Specificity = 99.6%; Concordance = 99.4% | | | | |

*L. pneumophila:*

| | | Multiplex Assay Results | | |
|---|---|---|---|---|
| | | Positive | Negative | Total |
| TaqMan™ Assay Results | Positive | 45 | 1 | 46 |
| | Negative | 0 | 265 | 265 |
| | Total | 45 | 266 | 311 |

| | | | | |
|---|---|---|---|---|
| Sensitivity = 97.8%; Specificity = 100%; Concordance = 99.7% | | | | |

It was further determined that the multiplex assay has a limit of detection of about 10-13 copies per reaction, and has a dynamic range of≥7 log dilutions.

### EXAMPLE 4: Cross-reactivity of the Multiplex Atypical Pneumonia Pathogen Assay.

DNA was extracted from the organisms listed below and assayed in the multiplex PCR system described above. None of the organisms showed significant cross-reactivity in the assay.

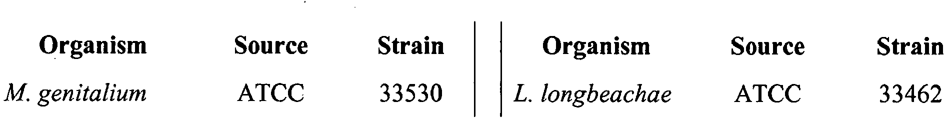

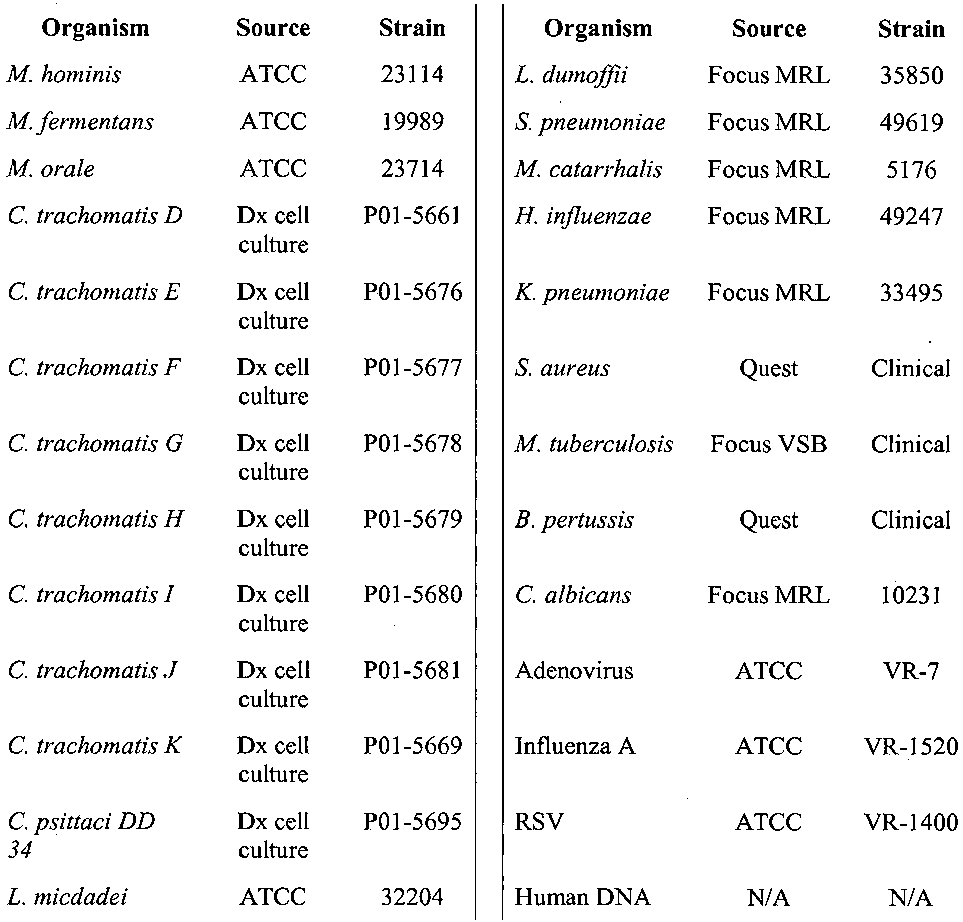

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention.

In addition, where features or aspects of the invention are described in terms of Markush groups, those skilled in the art will recognize that the invention is also thereby described in terms of any individual member or subgroup of members of the Markush group.

## Claims

1. A method for identifying the presence or absence of a pathogen in a biological sample, comprising detecting the presence or absence of a *Mycoplasma pneumoniae* P1 gene fragment comprising at least 15 nucleotides from the sequence of SEQ ID NO: 2, or a complement thereof.

2. The method of claim 1, further comprising detecting the presence or absence of a *Chlamydophila pneumoniae* Cpn0980 gene fragment comprising at least 15 nucleotides from the sequence of SEQ ID NO: 7, or a complement thereof.

3. The method of claim 1 or 2, further comprising detecting the presence or absence of a *Legionella pneumophila* pmiA gene fragment comprising at least 15 nucleotides from the sequence of SEQ ID NO: 12, or a complement thereof.

4. The method of claim 1, wherein
(1) said method comprises amplifying any two of said P1 gene or fragment thereof, Cpn0980 gene or fragment thereof, and pmiA gene or fragment thereof; and/or
(2) said method comprises:
(a) providing primer pairs suitable for amplifying in a single reaction, any two of:
(i) the *Mycoplasma pneumoniae* P1 gene fragment,
(ii) the *Chlamydophila pneumoniae* Cpn0980 gene fragment, and
(iii) the *Legionella pneumophila* pmiA gene fragment;
(b) contacting the biological sample with the primer pairs of step (a) under, conditions wherein amplification products are produced; and
(c) identifying a pathogen by detecting the amplification products produced in step (b); and/or
(3) the P1 gene fragment is amplified using at least one primer comprising the sequence of SEQ ID NOs: 4 and 5 or a complement thereof; and/or
(4) the P1 gene fragment is detected using a probe comprising the sequence of SEQ ID NO: 3; and/or
(5) the Cpn0980 gene fragment is amplified using at least one primer comprising the sequence of SEQ ID NOs: 9 and 10 or a complement thereof; and/or
(6) the Cpn0980 gene fragment is detected using a probe comprising the sequence of SEQ ID NO: 8; and/or
(7) the pmiA gene fragment is amplified using at least one primer comprising the sequence of SEQ ID NOs: 14-17 or a complement thereof; and/or
(8) the pmiA gene fragment is detected using a probe comprising the sequence of SEQ ID NO: 13; and/or
(9) said method comprises detecting the presence or absence of each of (a), (b), and (c); and/or
(10) at least one primer of each primer pair is provided as a Scorpion primer; and/or
(11) at least one primer of each primer pair is provided as a Scorpion primer and at least one of said Scorpion primers is selected from the group consisting of SEQ ID NOs: 18-25 and 45-48, or complements thereof.

5. The method of claims 1 or 2, wherein said method comprises detecting *Chlanaydophila pneumoniae* in a biological sample,
the method comprising detecting the presence of the Cpn0980 gene or a fragment comprising at least 15 nucleotides from the sequence of SEQ ID NO: 7, or the complement thereof, wherein
(1) said fragment of the Cpn0980 gene is optionally amplified using at least one primer comprising the sequence of SEQ ID NO: 9 and 10 or a complement thereof; and/or
(2) said fragment of the Cpn0980 gene is optionally detected using a probe comprising the sequence of SEQ ID NO 8.

6. The method of claim 1, wherein said method comprises detecting *Legionella pneumophila* in a biological sample, the method comprising detecting the presence of the pmiA gene fragment comprising at least 15 nucleotides from the sequence of SEQ ID NO: 12, or a complement thereof, wherein
(1) said fragment of the pmiA gene is optionally amplified using at least one primer comprising the sequence of SEQ ID NOs: 14-17 or a complement thereof; and/or
(2) said fragment of the pmiA gene is optionally detected using a probe comprising the sequence of SEQ ID NO: 13.

7. The method of claim 1, wherein said method comprises detecting *Mycoplasma pneumophila* in a biological sample, the method comprising detecting the presence of the P1 gene fragment comprising at least 15 contiguous nucleotides from the sequence of SEQ ID NO: 2, or a complement thereof, wherein
(1) said fragment of the P1 gene fragment is optionally amplified using at least one primer comprising the sequence of SEQ ID NOs 4 or 5, or a complement thereof; and/or
(2) said fragment of the pmiA gene is optionally detected using a probe comprising the sequence of SEQ ID NO: 3.

8. *An in vitro* method of diagnosing an individual for infection with an atypical pneumoniae organism, comprising evaluating a biological sample obtained from the individual for the presence or absence of a *Mycoplasma pneumoniae* P1 gene fragment comprising at least 15 nucleotides from the sequence of SEQ ID NO: 2, or a complement thereof,
wherein the presence of said gene or fragment indicates that the individual is infected with the associated organism.

9. The *in vitro* method of claim 8, further comprising evaluating a biological sample obtained from the individual for the presence or absence of a *Chlarnydophila pneumoniae* Cpn0980 gene fragment comprising at least 15 nucleotides from the sequence of SEQ ID NO: 7, or a complement thereof, wherein the presence of said gene or fragment indicates that the individual is infected with the associated organism.

10. The *in vitro* method of claims 8 or 9, further comprising evaluating a biological sample obtained from the individual for the presence or absence of a *Legionella pneumophila* pmiA gene fragment comprising at least 15 nucleotides from the sequence of SEQ ID NO: 12, or a complement thereof,
wherein the presence of said gene or fragment indicates that the individual is infected with the associated organism.

11. *The in vitro* method of claim 8, wherein
(1) said method comprises
(a) providing primer pairs suitable for amplifying in a single reaction, any two of:
(i) the *Mycoplasma pneumoniae* P1 gene fragment,
(ii) the *Chlamydophila pneumoniae* Cpn0980 gene fragment, and
(iii) the *Legionella pneumophila* pmiA gene fragment,
(b) contacting the biological sample with the primer pairs of step (a) under conditions wherein amplification products are produced, and
(c) identifying a pathogen by detecting the amplification products produced in step (b); and/or
(2) the P1 gene fragment comprises at least 15 nucleotides from the sequence of SEQ ID NO: 2, or a complement thereof; and/or
(3) the P1 gene fragment comprises at least 15 nucleotides from the sequence of SEQ ID NOs: 4 and 5, or a complement thereof; and/or
(4) the P1 gene fragment is detected using a probe comprising the sequence of SEQ ID NO: 3; and/or
(5) the Cpn0980 gene fragment comprises at least 15 nucleotides from the sequence of SEQ ID NO: 7; and/or
(6) the Cpn0980 fragment gene is amplified using at least one primer comprising the sequence of SEQ ID NOs: 9 and 10 or a complement thereof; and/or
(7) the Cpn0980 gene fragment is detected using a probe comprising the sequence of SEQ ID NO: 8; and/or
(8) the pmiA gene fragment comprises at least 15 nucleotides from the sequence of SEQ ID NO: 12; and/or
(9) the pmiA gene fragment is amplified using at least one primer comprising the sequence of SEQ ID NOs: 14-17 or a complement thereof; and/or
(10) the pmiA gene fragment is detected using a probe comprising the sequence of SEQ ID NO: 13; and/or
(11) the primers of each of (a)(i), (a)(ii), and (a)(iii) are provided; and/or
(12) at least one primer of each primer pair is provided as a Scorpion primer; and/or
(13) at least one primer of each primer pair is provided as a Scorpion primer and at least one of said Scorpion primers is selected from the group consisting of SEQ ID NOs: 18-25 and 45-48.

12. A kit comprising:
a pair of P1 primers that specifically hybridize to the P1 gene of *M. pneumoniae* and are capable of amplifying a P1 gene fragment, wherein the P1 primers specifically hybridize to a nucleic acid having the sequence of SEQ ID NO: 2; and a P1 probe capable of specifically hybridizing to the P1 fragment amplified by the P1 primers,
wherein optionally the P1 probe specifically hybridizes to a nucleic acid having the sequence of SEQ ID NO: 2.

13. The kit of claim 12, further comprising a pair of Cpn0980 primers that specifically hybridize to the Cpn0980 gene of *C*. *pneumoniae* and are capable of amplifying a Cpn0980 gene fragment, and a Cpn0980 probe capable of specifically hybridizing to the Cpn0980 fragment amplified by the Cpn0980 primers;
wherein the Cpn0980 primers specifically hybridize to a nucleic acid having the sequence of SEQ ID NO: 6; and wherein
the Cpn0980 probe specifically hybridizes to a nucleic acid having the sequence of SEQ ID NO: 6.

14. The kit of claims 12 or 13, further comprising a pair of pmiA primers that specifically hybridize to the pmiA gene of *L. pneumophila* and are capable of amplifying a pmiA gene fragment, and a pmiA probe that specifically hybridizes to the pmiA gene fragment amplified by the pmiA primers,
wherein the pmiA primers specifically hybridize to a nucleic acid having the sequence of SEQ ID NO: 11; and wherein
the pmiA probe specifically hybridizes to a nucleic acid having the sequence of SEQ ID NO: 11.

15. The kit of claim 14, wherein said kit comprises said pair of P1 primers, said pair of Cpn0980 primers, and said pair of pmiA primers.

## Patentansprüche

1. Verfahren zum Identifizieren der Anwesenheit oder der Abwesenheit eines Pathogenes in einer biologischen Probe, umfassend Detektion der Anwesenheit oder Abwesenheit eines *Mycoplasma pneumoniae* P1-Genfragments, das mindestens 15 Nukleotide von der Sequenz von SEQ ID NO: 2 umfasst, oder eines Komplements davon.

2. Verfahren gemäß Anspruch 1, weiterhin umfassend Detektion der Anwesenheit oder Abwesenheit eines *Chlamydophila pneumoniae* Cpn0980-Genfragments, das mindestens 15 Nukleotide von der Sequenz von SEQ ID NO: 7 umfasst, oder eines Komplements davon.

3. Verfahren gemäß Anspruch 1 oder 2, weiterhin umfassend Detektion der Anwesenheit oder Abwesenheit eines *Legionella pneumophila* pmiA-Genfragments, das mindestens 15 Nukleotide von der Sequenz von SEQ ID NO: 12 umfasst, oder eines Komplements davon.

4. Verfahren gemäß Anspruch 1, wobei
(1) das Verfahren das Amplifizieren von zwei beliebigen des genannten P1-Gens oder dessen Fragmente, des Cpn0980 Gens oder dessen Fragmente, und des pmiA-Gens oder dessen Fragmente umfasst; und/oder
(2) das Verfahren umfasst:
(a) Bereitstellen von Primerpaaren, die geeignet sind für das Amplifizieren in einer einzelnen Reaktion von zwei beliebigen von:
(i) dem *Mycoplasma pneumoniae* P1-Genfragment,
(ii) dem *Chlamydophila pneumoniae* Cpn0980-Genfragment, und
(iii) dem *Legionella pneumophila* pmiA-Genfragment;
(b) Kontaktieren der biologischen Probe mit dem Primerpaar aus Schritt (a) unter Bedingungen, bei denen Amplifikationsprodukte erzeugt werden; und
(c) Identifizieren eines Pathogenes durch Detektion der Amplifikationsprodukte, die in Schritt (b) erzeugt werden; und/oder
(3) das P1-Genfragment amplifiziert wird unter Verwendung mindestens eines Primers, das die Sequenz von SEQ ID NOs: 4 und 5 umfasst, oder eines Komplements davon, und/oder
(4) das P1-Genfragment detektiert wird unter Verwendung einer Sonde, welches die Sequenz von SEQ ID NO: 3 umfasst; und/oder
(5) das Cpn0980-Genfragment amplifiziert wird unter Verwendung mindestens eines Primers, das die Sequenz von SEQ ID NOs: 9 und 10 umfasst, oder eines Komplements davon, und/oder
(6) das Cpn0980-Genfragment detektiert wird unter Verwendung einer Sonde, welche die Sequenz von SEQ ID NO: 8 umfasst; und/oder
(7) das pmiA-Genfragment amplifiziert wird unter Verwendung mindestens eines Primers, das die Sequenz von SEQ ID NOs: 14-17 umfasst, oder eines Komplements davon, und/oder
(8) das pmiA-Genfragment detektiert wird unter Verwendung einer Sonde, welches die Sequenz von SEQ ID NO: 13 umfasst; und/oder
(9) das Verfahren das Detektieren der Anwesenheit oder Abwesenheit jedes der (a), (b), und (c) umfasst, und/oder
(10) mindestens ein Primer aus jedem Primerpaar als *Scorpion-Primer* bereitgestellt wird; und/oder
(11) mindestens ein Primer aus jedem Primerpaar als *Scorpion-Primer* bereitgestellt wird und mindestesns ein *Scorpion-Primer* ausgewählt ist aus der Gruppe bestehend aus SEQ ID NOs: 18-25 und 45-48, oder Komplemente davon.

5. Verfahren gemäß Anspruch 1 oder 2, wobei das Verfahren das Detektieren von *Chlamydophila pneumoniae* in einer biologischen Probe umfasst,
das Verfahren umfassend Detektion der Anwesenheit des Cpn0980-Gens oder eines Fragments, das mindestens 15 Nukleotide von der Sequenz von SEQ ID NO: 7 umfasst, oder eines Komplements davon, wobei
(1) das genannte Fragment des Cpn0980-Gens optional unter Verwendung mindestens eines Primers amplifiziert wird, das die Sequenz von SEQ ID NOs: 9 und 10 umfasst, oder ein Komplement davon; und/oder
(2) das Fragment des Cpn0980-Gens optional detektiert wird unter Verwendung einer Sonde, welche die Sequenz von SEQ ID NO 8 umfasst.

6. Verfahren nach Anspruch 1, wobei das Verfahren das Detektieren von *Legionella pneumophila* in einer biologischen Probe umfasst, das Verfahren umfassend Detektieren der Anwesenheit des pmiA-Genfragments, das mindestens 15 Nukleotide von der Sequenz von SEQ ID NO: 12 umfasst, oder eines Komplements davon, wobei
(1) das Fragment des pmiA-Gens optional unter Verwendung mindestens eines Primers amplifiziert wird, das die Sequenz von SEQ ID NOs: 14-17 umfasst, oder eines Komplements davon; und/oder
(2) das Fragment des pmiA-Gens optional detektiert wird unter Verwendung einer Sonde, welche die Sequenz von SEQ ID NO 13 umfasst.

7. Verfahren gemäß Anspruch 1, wobei das Verfahren das Detektieren von *Mycoplasma pneumophila* in einer biologischen Probe umfasst, das Verfahren umfassend Detektieren der Anwesenheit des P1-Genfragments, das mindestens 15 zusammenhängende Nukleotide von der Sequenz von SEQ ID NO: 2 umfasst, oder eines Komplements davon, wobei
(1) das Fragment des P1-Genfragments optional unter Verwendung mindestens eines Primers amplifiziert wird, das die Sequenz von SEQ ID NOs: 4 oder 5 umfasst, oder eines Komplements davon; und/oder
(2) das Fragment des pmiA-Gens optional detektiert wird unter Verwendung einer Sonde, welche die Sequenz von SEQ ID NO 3 umfasst.

8. In-vitro Verfahren zur Diagnose einer Infektion mit einem atypischen *pneumoniae-*Organismus bei einem Individuum, umfassend Auswerten einer biologischen Probe, die von dem Individuum erhalten wurde, nach der Anwesenheit oder Abwesenheit eines *Mycoplasma pneumoniae* P1-Genfragments, das mindestens 15 Nukleotide von der Sequenz von SEQ ID NO: 2 umfasst, oder eines Komplements davon,
wobei die Anwesenheit des Gens oder Fragments indiziert, dass das Individuum mit dem zugehörigen Organismus infiziert ist.

9. In-vitro Verfahren gemäß Anspruch 8, weiterhin umfassend Auswerten einer biologischen Probe, die von dem Individuum erhalten wurde, nach der Anwesenheit oder Abwesenheit eines *Chlamydophila pneumoniae* Cpn0980-Genfragments, das mindestens 15 Nukleotide von der Sequenz von SEQ ID NO: 7 umfasst, oder eines Komplements davon, wobei die Anwesenheit des Gens oder Fragments indiziert, dass das Individuum mit dem zugehörigen Organismus infiziert ist

10. In-vitro Verfahren gemäß Anspruch 8 oder 9, weiterhin umfassend Auswerten einer biologischen Probe, die von dem Individuum erhalten wurde, nach der Anwesenheit oder Abwesenheit eines *Legionella pneumophila* pmiA-Genfragments, das mindestens 15 Nukleotide von der Sequenz von SEQ ID NO: 12 umfasst, oder eines Komplements davon,
wobei die Anwesenheit des Gens oder Fragments indiziert, dass das Individuum mit dem zugehörigen Organismus infiziert ist

11. In-vitro Verfahren gemäß Anspruch 8, wobei
(1) das Verfahren umfasst
(a) Bereitstellen von Primerpaaren, die geeignet sind für das Amplifizieren in einer einzelnen Reaktion von zwei beliebigen von:
(i) dem *Mycoplasma pneumoniae* P1-Genfragment,
(ii) dem *Chlamydophila pneumoniae* Cpn0980-Genfragment, und
(iii) dem *Legionella pneumophila* pmiA-Genfragment;
(b) Kontaktieren der biologischen Probe mit dem Primerpaar aus Schritt (a) unter Bedingungen, bei denen Amplifikationsprodukte erzeugt werden; und
(c) Identifizieren eines Pathogenes durch Detektieren der Amplifikationsprodukte, die in Schritt (b) erzeugt werden; und/oder
(2) das P1-Genfragment umfasst mindestens 15 Nukleotide von der Sequenz von SEQ ID NO: 2, oder ein Komplement davon; und/oder
(3) das P1-Genfragment umfasst mindestens 15 Nukleotide von der Sequenz von SEQ ID NOs: 4 and 5, oder ein Komplement davon; und/oder
(4) das P1-Genfragment detektiert wird unter Verwendung einer Sonde, welches die Sequenz von SEQ ID NO: 3 umfasst; und/oder
(5) das Cpn0980-Genfragment umfasst mindestens 15 Nukleotide von der Sequenz von SEQ ID NO: 7; und/oder
(6) das Cpn0980-Genfragment amplifiziert wird unter Verwendung mindestens eines Primers, das die Sequenz von SEQ ID NOs: 9 und 10, oder eines Komplements davon umfasst; und/oder
(7) das Cpn0980-Genfragment detektiert wird unter Verwendung einer Sonde, welche die Sequenz von SEQ ID NO: 8 umfasst; und/oder
(8) das pmiA -Genfragment mindestens 15 Nukleotide von der Sequenz von SEQ ID NO: 12 umfasst; und/oder
(9) das pmiA-Genfragment amplifiziert wird unter Verwendung mindestens eines Primers, das die Sequenz von SEQ ID NOs: 14-17, oder eines Komplements davon umfasst, und/oder
(10) das pmiA-Genfragment detektiert wird unter Verwendung einer Sonde, welches die Sequenz von SEQ ID NO: 13 umfasst; und/oder
(11) die Primer jedes der (a)(i), (a)(ii), and (a)(iii) bereitgestellt werden; und/oder
(12) mindestens ein Primer aus jedem Primerpaar als *Scorpion-Primer* bereitgestellt wird; und/oder
(13) mindestens ein Primer aus jedem Primerpaar als *Scorpion-Primer* bereitgestellt wird und mindestens ein *Scorpion-Primer* ausgewählt ist aus der Gruppe bestehend aus SEQ ID NOs: 18-25 und 45-48.

12. Kit umfassend:
ein Paar von P1-Primern, die spezifisch mit dem P1-Gen von *M. pneumoniae* hybridisieren, und die in der Lage sind, ein P1-Genfragment zu amplifizieren, wobei die P1-Primer spezifisch mit einer Nukleinsäure hybridisieren, die eine Sequenz von SEQ ID NO: 2 hat; und eine P1-Sonde, die in der Lage ist, spezifisch mit dem P1-Fragment, das mit den P1-Primern amplifiziert wurde, zu hybridisieren, wobei optional die P1-Sonde spezifisch mit einer Nukleinsäure hybridisiert, die die Sequenz von SEQ ID NO: 2 hat.

13. Kit gemäß Anspruch 12, weiterhin umfassend ein Paar von Cpn0980-Primern, die spezifisch mit dem Cpn0980-Gen von *C. pneumoniae* hybridisieren, und die in der Lage sind, ein Cpn0980-Genfragment zu amplifizieren, und eine Cpn0980-Sonde, die in der Lage ist, spezifisch mit dem Cpn0980-Fragment, das mit den Cpn0980-Primern amplifiziert wurde, zu hybridisieren,
wobei die Cpn0980-Primer spezifisch mit einer Nukleinsäure hybridisieren, die eine Sequenz von SEQ ID NO: 6 hat; und wobei
die Cpn0980-Sonde spezifisch mit einer Nukleinsäure hybridisiert, die die Sequenz von SEQ ID NO: 6 hat.

14. Kit gemäß Anspruch 12 oder 13, weiter umfassend ein Paar von pmiA-Primern, die spezifisch mit dem pmiA-Gen von *L. pneumophila* hybridisieren, und die in der Lage sind, ein pmiA-Genfragment zu amplifizieren, und eine pmiA-Sonde, die spezifisch mit dem pmiA-Genfragment hybridisiert, das mit den pmiA-Primern amplifiziert wurde, wobei die pmiA-Primer spezifisch mit einer Nukleinsäure hybridisieren, die eine Sequenz von SEQ ID NO: 11 hat; und wobei
die pmiA-Sonde spezifisch mit einer Nukleinsäure hybridisiert, die die Sequenz von SEQ ID NO: 11 hat.

15. Kit gemäß Anspruch 14, wobei das Kit genanntes Paar von P1-Primern, genanntes Paar von Cpn0980-Primern und genanntes Paar von pmiA-Primern umfasst.

## Revendications

1. Procédé d'identification de la présence ou absence d'un pathogène dans un échantillon biologique, comprenant détecter la présence ou absence d'un fragment de gène P1 de Mycoplasma pneumoniae comprenant au moins 15 nucléotides provenant de la séquence de SEQ ID N° : 2, ou d'un complément de celle-ci.

2. Procédé selon la revendication 1, comprenant en outre détecter la présence ou absence d'un fragment de gène Cpn0980 de Chlamydophila pneumoniae comprenant au moins 15 nucléotides provenant de la séquence de SEQ ID N° : 7, ou d'un complément de celle-ci.

3. Procédé selon la revendication 1 ou 2, comprenant en outre détecter la présence ou absence d'un fragment de gène pmiA de Legionella pneumophila comprenant au moins 15 nucléotides provenant de la séquence de SEQ ID N° : 12, ou d'un complément de celle-ci.

4. Procédé selon la revendication 1, dans lequel
(1) ledit procédé comprend amplifier toute paire parmi ledit gène P1 ou fragment de celui-ci, gène Cpn0980 ou fragment de celui-ci et gène pmiA ou fragment de celui-ci ; et/ou
(2) ledit procédé comprend :
(a) fournir des paires d'amorces convenant à l'amplification dans une seule réaction, toute paire parmi :
(i) le fragment de gène P1 de Mycoplasma pneumoniae,
(ii) le fragment de gène Cpn0980 de Chlamydophila pneumoniae, et
(iii) le fragment de gène pmiA de Legionella pneumophila ;
(b) mettre en contact l'échantillon biologique avec les paires d'amorces de l'étape (a) dans des conditions dans lesquelles les produits d'amplification sont produits ; et
(c) identifier un pathogène en détectant les produits d'amplification produits à l'étape (b) ; et/ou
(3) le fragment de gène P1 est amplifié en utilisant au moins une amorce comprenant la séquence de SEQ ID N° : 4 et 5 ou un complément de celle-ci ; et/ou
(4) le fragment de gène P1 est détecté en utilisant une sonde comprenant la séquence de SEQ ID N° : 3 ; et/ou
(5) le fragment de gène Cpn0980 est amplifié en utilisant au moins une amorce comprenant la séquence de SEQ ID N° : 9 et 10 ou un complément de celle-ci ; et/ou
(6) le fragment de gène Cpn0980 est détecté en utilisant une sonde comprenant la séquence de SEQ ID N° : 8 ; et/ou
(7) le fragment de gène pmiA est amplifié en utilisant au moins une amorce comprenant la séquence de SEQ ID N° : 14 à 17 ou un complément de celle-ci ; et/ou
(8) le fragment de gène pmiA est détecté en utilisant une sonde comprenant la séquence de SEQ ID N° : 13 ; et/ou
(9) ledit procédé comprend détecter la présence ou absence de chacun de (a), (b) et (c) ; et/ou
(10) au moins une amorce de chaque paire d'amorces est fournie en tant qu'amorce Scorpion ; et/ou
(11) au moins une amorce de chaque paire d'amorces est fournie en tant qu'amorce Scorpion et au moins une desdites amorces Scorpion est sélectionnée parmi le groupe constitué de SEQ ID N° : 18 à 25 et 45 à 48, des compléments de celles-ci.

5. Procédé selon la revendication 1 ou 2, dans lequel le procédé comprend détecter Chlamydophila pneumoniae dans un échantillon biologique,
le procédé comprenant détecter la présence du gène Cpn0980 ou d'un fragment comprenant au moins 15 nucléotides provenant de la séquence de SEQ ID N° : 7, ou du complément de celle-ci, dans lequel
(1) ledit fragment du gène Cpn0980 est amplifié en option en utilisant au moins une amorce comprenant la séquence de SEQ ID N° : 9 et 10 ou un complément de celle-ci ; et/ou
(2) ledit fragment du gène Cpn0980 est détecté en option en utilisant une sonde comprenant la séquence de SEQ ID N° 8.

6. Procédé selon la revendication 1, dans lequel ledit procédé comprend détecter Legionella pneumophila dans un échantillon biologique, le procédé comprenant détecter la présence du fragment de gène pmiA comprenant au moins 15 nucléotides provenant de la séquence de SEQ ID N° : 12, ou d'un complément de celle-ci, dans lequel
(1) ledit fragment du gène pmiA est amplifié en option en utilisant au moins une amorce comprenant la séquence de SEQ ID N° : 14 à 17 ou un complément de celle-ci ; et/ou
(2) ledit fragment du gène pmiA est détecté en option en utilisant une sonde comprenant la séquence de SEQ ID N° : 13.

7. Procédé selon la revendication 1, dans lequel ledit procédé comprend détecter Mycoplasma pneumophila dans un échantillon biologique, le procédé comprenant détecter la présence du fragment de gène P1 comprenant au moins 15 nucléotides contigus provenant de la séquence de SEQ ID N° : 2, ou d'un complément de celle-ci, dans lequel
(1) ledit fragment du fragment de gène P1 est amplifié en option en utilisant au moins une amorce comprenant la séquence de SEQ ID N° 4 ou 5, ou un complément de celle-ci ; et/ou
(2) ledit fragment du gène pmiA est détecté en option en utilisant une sonde comprenant la séquence de SEQ ID N° : 3.

8. Procédé in vitro de diagnostic d'un individu pour une infection avec un organisme de pneumoniae atypique, comprenant évaluer un échantillon biologique obtenu auprès de l'individu pour la présence ou absence d'un fragment de gène P1 de Mycoplasma pneumoniae comprenant au moins 15 nucléotides provenant de la séquence de SEQ ID N° : 2, ou d'un complément de celle-ci,
dans lequel la présence dudit gène ou fragment indique que l'individu est infecté avec l'organisme associé.

9. Procédé in vitro selon la revendication 8, comprenant en outre évaluer un échantillon biologique obtenu auprès de l'individu pour la présence ou absence d'un fragment de gène Cpn0980 de Chlamydophila pneumoniae comprenant au moins 15 nucléotides provenant de la séquence de SEQ ID N° : 7, ou d'un complément de celle-ci, dans lequel la présence dudit gène ou fragment indique que l'individu est infecté avec l'organisme associé.

10. Procédé in vitro selon la revendication 8 ou 9, comprenant en outre évaluer un échantillon biologique obtenu auprès de l'individu pour la présence ou absence d'un fragment de gène pmiA de Legionella pneumophila comprenant au moins 15 nucléotides provenant de la séquence de SEQ ID N° : 12, ou d'un complément de celle-ci,
dans lequel la présence dudit gène ou fragment indique que l'individu est infecté avec l'organisme associé.

11. Procédé in vitro selon la revendication 8, dans lequel
(1) ledit procédé comprend
(a) fournir des paires d'amorces convenant à l'amplification dans une seule réaction, toute paire parmi :
(i) le fragment de gène P1 de Mycoplasma pneumoniae,
(ii) le fragment de gène Cpn0980 de Chlamydophila pneumoniae, et
(iii) le fragment de gène pmiA de Legionella pneumophila,
(b) mettre en contact l'échantillon biologique avec les paires d'amorces de l'étape (a) dans des conditions dans lesquelles les produits d'amplification sont produits, et
(c) identifier un pathogène en détectant les produits d'amplification produits à l'étape (b) ; et/ou
(2) le fragment de gène P1 comprend au moins 15 nucléotides provenant de la séquence de SEQ ID N° : 2, ou d'un complément de celle-ci ; et/ou
(3) le fragment de gène P1 comprend au moins 15 nucléotides provenant de la séquence de SEQ ID N° : 4 et 5, ou d'un complément de celle-ci ; et/ou
(4) le fragment de gène P1 est détecté en utilisant une sonde comprenant la séquence de SEQ ID N° : 3 ; et/ou
(5) le fragment de gène Cpn0980 comprend au moins 15 nucléotides provenant de la séquence de SEQ ID N° : 7 ; et/ou
(6) le fragment de gène Cpn0980 est amplifié en utilisant au moins une amorce comprenant la séquence de SEQ ID N° : 9 et 10 ou un complément de celle-ci ; et/ou
(7) le fragment de gène Cpn0980 est détecté en utilisant une sonde comprenant la séquence de SEQ ID N° : 8 ; et/ou
(8) le fragment de gène pmiA comprend au moins 15 nucléotides provenant de la séquence de SEQ ID N° : 12 ; et/ou
(9) le fragment de gène pmiA est amplifié en utilisant au moins une amorce comprenant la séquence de SEQ ID N° : 14 à 17 ou un complément de celle-ci ; et/ou
(10) le fragment de gène pmiA est détecté en utilisant une sonde comprenant la séquence de SEQ ID N° : 13 ; et/ou
(11) les amorces de chacun de (a)(i), (a)(ii) et (a) (iii) sont fournies ; et/ou
(12) au moins une amorce de chaque paire d'amorces est fournie en tant qu'amorce Scorpion ; et/ou
(13) au moins une amorce de chaque paire d'amorces est fournie en tant qu'amorce Scorpion et au moins une desdites amorces Scorpion est sélectionnée parmi le groupe constitué de SEQ ID N° : 18 à 25 et 45 à 48.

12. Kit comprenant :
une paire d'amorces P1 qui s'hybrident spécifiquement au gène P1 de M. pneumoniae et sont capables d'amplifier un fragment de gène P1, dans lequel les amorces P1 s'hybrident spécifiquement à un acide nucléique ayant la séquence de SEQ ID N° : 2 ; et une sonde P1 capable de s'hybrider spécifiquement au fragment P1 amplifié par les amorces P1,
dans lequel la sonde P1 s'hybride spécifiquement en option à un acide nucléique ayant la séquence de SEQ ID N° : 2.

13. Kit selon la revendication 12, comprenant en outre une paire d'amorces Cpn0980 qui s'hybrident spécifiquement au gène Cpn0980 de C. pneumoniae et sont capables d'amplifier un fragment de gène Cpn0980, et une sonde Cpn0980 capable de s'hybrider spécifiquement au fragment Cpn0980 amplifié par les amorces Cpn0980 ;
dans lequel les amorces Cpn0980 s'hybrident spécifiquement à un acide nucléique ayant la séquence de SEQ ID N° : 6 ; et dans lequel
la sonde Cpn0980 s'hybride spécifiquement à un acide nucléique ayant la séquence de SEQ ID N° : 6.

14. Kit selon la revendication 12 ou 13, comprenant en outre une paire d'amorces pmiA qui s'hybrident spécifiquement au gène pmiA de L. pneumophila et sont capables d'amplifier un fragment de gène pmiA, et une sonde pmiA qui s'hybride spécifiquement au fragment de gène pmiA amplifié par les amorces pmiA,
dans lequel les amorces pmiA s'hybrident spécifiquement à un acide nucléique ayant la séquence de SEQ ID N° : 11 ; et dans lequel
la sonde pmiA s'hybride spécifiquement à un acide nucléique ayant la séquence de SEQ ID N° : 11.

15. Kit selon la revendication 14, dans lequel ledit kit comprend ladite paire d'amorces P1, ladite paire d'amorces Cpn0980 et ladite paire d'amorces pmiA.
